# FASCICULE DE BREVET EUROPEEN

(11) **EP 1 143 971 B1**
(45) Date de publication et mention de la délivrance du brevet: **02.06.2004**
(21) Numéro de dépôt: 00900638.8
(22) Date de dépôt: 21.01.2000
(51) Int. Cl.: A61K 31/445, C07D 211/58

(54) **1-(PIPERIDIN-4-YL)-3-(ARYL)-ISOTHIOUREES SUBSTITUEES, LEUR PREPARATION ET LEUR APPLICATION EN THERAPEUTIQUE**
SUBSTITUIERTE 1-(PIPERIDIN-4-YL)-3-(ARYL)-ISOTHIOHARNSTOFFE , IHRE HERSTELLUNG UND THERAPEUTISCHE VERWENDUNG
SUBSTITUTED 1-(PIPERIDIN-4-YL)-3-(ARYL)-ISOTHIOUREAS, THEIR PREPARATION AND THERAPEUTIC USE

(30) Priorité: 22.01.1999 FR 9900705
(43) Date de publication de la demande: 17.10.2001
(73) Titulaire: PIERRE FABRE MEDICAMENT, 92100 Boulogne-Billancourt (FR)
(72) Inventeur: PATOISEAU, Jean-François, F-81100 Castres (FR); RIEU, Jean-Pierre, F-81100 Castres (FR); JOHN, Gareth, F-81100 Castres (FR); LEGRAND, Bruno, F-81440 Lautrec (FR); VERSCHEURE, Yvan, F-81100 Castres (FR)
(74) Mandataire: Ahner, Francis
(86) Numéro de dépôt international: PCT/FR2000/000136
(87) Numéro de publication internationale: WO 2000/043011

(56) Documents cités:
- WO-A-97/05134

## Description

L'invention concerne de nouvelles 1,2-alcoyl-1-[1-[aryl(alcoyl)oxyalcoyl]pipéridin-4-yl]-3-aryl isothiourées substituées, leur procédé de préparation, ainsi que leur utilisation à titre de médicament.

Dans un brevet précédent de la demanderesse (WO-97.05134), il était revendiqué des dérivés de N-alcoyl-N-[1-(ω-aryloxyalcoyl)pipéridin-4-yl]-4H-3,1-benzothiazin-2-amines possédant un intérêt thérapeutique, particulièrement dans le traitement de l'ischémie myocardique.

De façon à induire une plus grande flexibilité de la molécule active, susceptible d'augmenter sa biodisponibilité et sa solubilité, les composés "ouverts" de la série précédente des benzothiazines ont été synthétisés et ont permis d'identifier une nouvelle classe de composés : les 3-aryl-1-(pipéridin-4-yl)-1-alcoyl isothiourées substituées objet de la présente invention. Leur étude pharmacologique a montré généralement une activité supérieure à la série cyclique de base sur le test in vitro de contracture à la vératrine de l'oreillette gauche isolée de rat ainsi que dans le test d'ischémie sur le coeur isolé perfusé du cobaye.

### MOLECULES REVENDIQUEES :

Les molécules de la présente invention appartiennent à la classe des 1-(1-aryl(alcoyl) oxyalcoylpiréridin-4-yl)-3-aryl isothiourées N et S substituées de formule I : 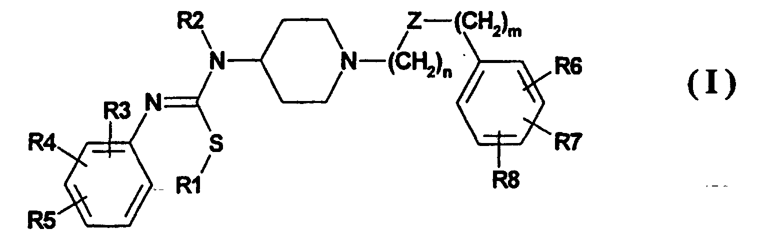 dans laquelle :
**R**_{**1**} et **R**_{**2**} égaux ou différents représentent un radical alcoyle saturé ou non, ramifié ou non renfermant de 1 à 7 atomes de carbone.
**R**_{**3**} à **R**_{**8**} égaux ou différents représentent :
   - un hydrogène
   - un alcoyle ramifié ou non renfermant de 1 à 5 atomes de carbone
   - un alcoyloxy ramifié ou non renfermant de 1 à 5 atomes de carbone
   - un groupement halogéno
   - un groupement nitro
   - un groupement hydroxy
   - un groupement acyle ou acyloxy renfermant de 2 à 5 atomes de carbone
   - un groupement dialcoylamino renfermant de 1 à 5 atomes de carbone
   - un groupement trifluorométhyle ou trifluorométhoxyle.
**Z** représente un atome d'oxygène ou de soufre ou un méthylène
**m** représente un nombre entier variant de 0 à 4 inclus.
**n** représente un nombre entier entre 2 et 7 inclus.

L'invention concerne aussi bien - lorsqu'ils existent - les isomères R ou S purs ou leurs mélanges.

La présente invention inclut les sels minéraux ou organiques, thérapeutiquement acceptables des composés de formule **I** et leurs hydrates éventuels.

L'invention concerne aussi le procédé de préparation des composés revendiqués, ainsi que leur application comme médicaments.

Les molécules de la présente invention possèdent des propriétés cytoprotectrices remarquables, supérieures à celles de la série cyclique de la famille des 4H-3,1-benzothiazin-2-amines et aux produits de référence tel que R56865.

### SYNTHESE DES COMPOSES DE FORMULE I :

Les 1,2-dialcoyl-1-[1-[aryl(alcoyl)oxyalcoyl] pipéridin-4-yl]-3-aryl thiourées (**I**) sont préparées en deux étapes à partir des N-alcoyl-N-[1-(aryl)oxyalcoyl pipéridin-4-yl] amines (**II**). La condensation de ces amines (**II**) sur un aryl isothiocyanate (**III**) selon la méthode de **Kaye** and **Parris** (*J. Org. Chem.* 1951, *16*, 1859-1863) fournit la thio urée correspondante (**IV**).

La S-alcoylation de celle-ci est réalisée par condensation d'un halogèno alcane ou un sulfate de dialcoyle, soit en adaptant la méthode de **Dupin S.** et **Pesson M.** (*Bull. Soc. Chim. Fr*., 1963, 144-150), soit en utilisant de l'oxyde de calcium pour bloquer les iodures formés en extrapolant la méthode de **Honkenen E.** et col *(J. Med. Chem*., 1983, *26*, 1433-38), pour donner directement le composé **I** de la présente invention (cf. **schéma 1).**

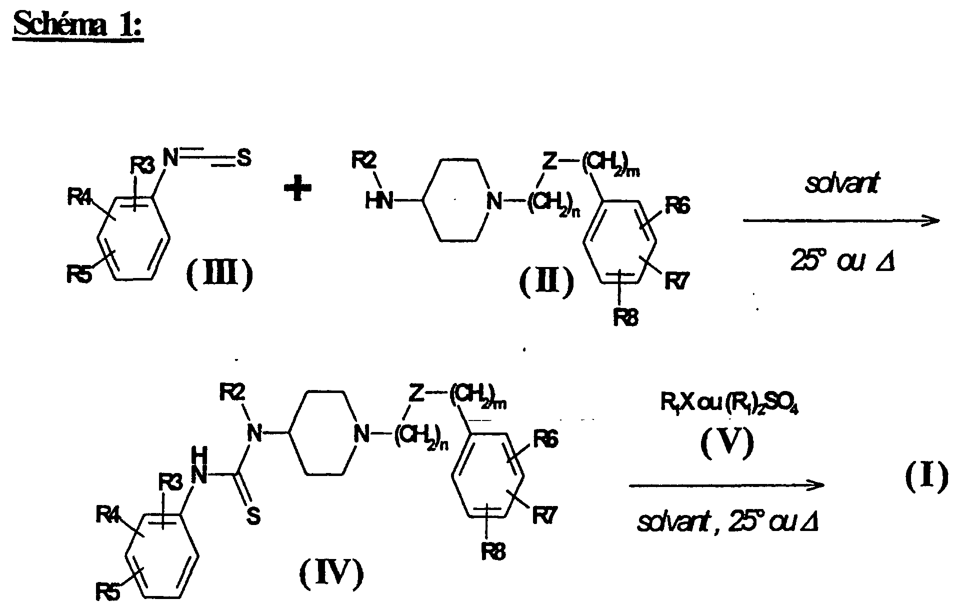

Dans le cas ou m=0 **(schéma 2)** les amines intermédiaires (**II**) ont été préparées selon **Ismaiel A.M** (*J. Med. Chem*., 1993,*36*, 2519-2525) comme décrit précé-demment dans le brevet WO 97.05134 en utilisant le triacétoxy borohydrure de sodium comme agent réducteur dans la dernière étape selon **Abdel-Magid A.F.** et coll. (*J*. *Org*. *Chem*. 1996, *61*, 3849-3862).

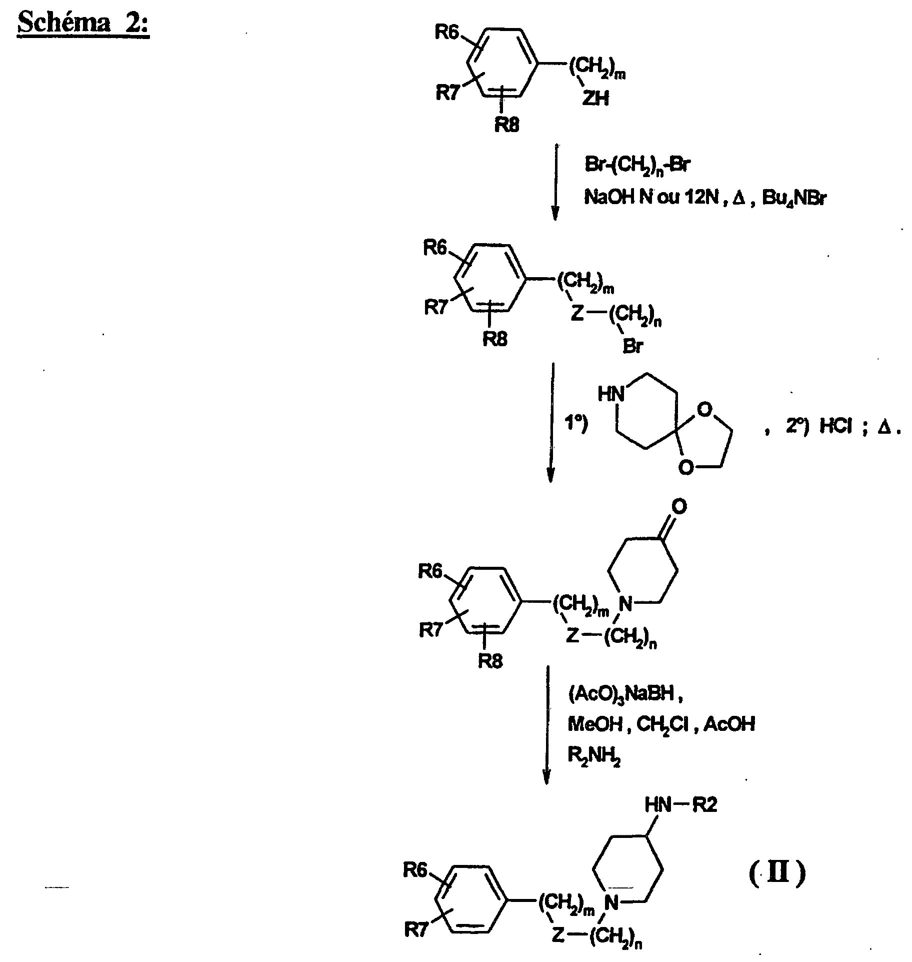

Si m ≠ 0, la réaction d'éthérification (première étape schéma 2) peut être réalisée à partir de l'alcool correspondant avec de la lessive de soude à 50 % par CTP selon **Burgstahler** et col, *J. Org. Chem.,* 1977, *42*, 566-8.

Les isothiocyanates de phényle non commerciaux sont préparés à partir des anilines correspondantes en utilisant le thiocarbonyl diimidazole comme décrit par **Staab H.A.** and **Walther G**., (*Liebigs Ann. Chem*., 1962, *567*, 104- ).

### Exemple 1:

### Hydrogénofumarate de la 1,2-diméthyl-3-phényl-1-[1-[4-(3,4-difluorophénoxy) butyl] piperidin-4-yl] isothiourée (1).

### 1.1)

1-Méthyl-3-phényl-1-[1-[4-(3,4-difluorophénoxy) butyl]pipéridin-4-yl] thiourée (**1.1**). Un mélange formé de 2g (14,8 mmol) d'isothiocyanate de phényle, 5,5 g (14,8 mmol) de dichlorhydrate de N-méthyl-1-[4-(3,4-difluorophénoxy)butyl] pipéridin-4-yl amine (cf. WO 97.05134) et 4,1 ml (29,6 mmol) de triéthylamine dans 25 ml d'éthanol, est porté au reflux pendant 2h. Après retour à 25 °C, le mélange est évaporé à siccité, repris dans 30 ml d'eau et extrait au chlorure de méthylène. La phase organique est lavée à l'eau, à l'eau salée puis séchée, sur sulfate de sodium anhydre. Après élimination de l'insoluble minéral, le filtrat est évaporé à sec pour donner un solide crème (m=5,9 g) qui, trituré dans l'alcool isopropylique donne 5,3 g (Rdt : 83 %) de poudre blanc cassé de formule 1.1. 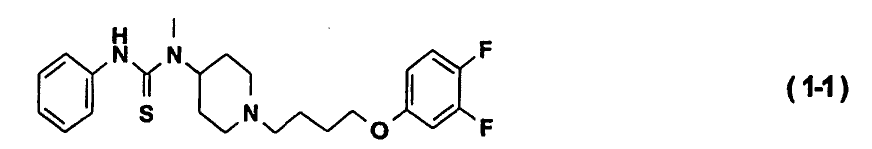 Formule brute : C₂₃H₂₉F₂N₃OS
Masse moléculaire : 433,54
Point de fusion : 152-3 °C
**RMN** (DMSO d₆) δ : 1,4-1,85 (m, 8H) ; 1,93 (m, 2H) ; 2,33 (m, 2H) ; 2,95 (m, 2H) ; 3,03 (s, 3H) ; 3,98 (t, 2H) ; 5,04 (m, 1 H) ; 6,7-6,9 (m, 1H) ; 7,02-7,2 (m, 2H) ; 7,25-7,4 (m,u5H) ; 9 (s, 1H).

**1.2)** Hydrogénofumarate de la 1,2-diméthyl-3-phényl-1-[1-[4-(3,4-difluorophénoxy) butyl] piperidin-4-yl] isothiourée (1). Dans un ballon de 100 ml, une solution de 2 g (4,6 mmol) d'urée précédente (1.1) dans un mélange de 30 ml de dichlorométhane et 40 ml d'éthanol est traitée avec 302 µl, (0,69 g ou 4,84 mmol) d'iodure de méthyle et agitée une nuit à 25 °C. Le mélange est évaporé à siccité, repris dans du dichlorométhane et de l'eau. La phase organique est séparée, lavée à l'eau salée et séchée sur sulfate de sodium anhydre. L'insoluble minéral est filtré, éliminé et le filtrat est évaporé à siccité : huile jaune (2 g). Cette huile est purifiée par flash-chromatographie en éluant avec un mélange CH₂Cl₂/CH₃OH/NH₄OH 97,5/2,25/0,25. On évapore les fractions renfermant la thiourée attendue : huile jaune clair (m=2,1 g ; Rdt : 66 %).

Hydrogénofumarate : 1,07 g de base dans 20 ml EtOH est agité à 25 °C ; le léger insoluble est éliminé par filtration puis 278 mg d'acide fumarique dans 5 ml d'alcool chaud sont ajoutés au filtrant précédent. Après cristallisation lente le sel organique de **formule 1** est collecté par filtration et séché (m=825 mg ; Rdt : 40 %) : 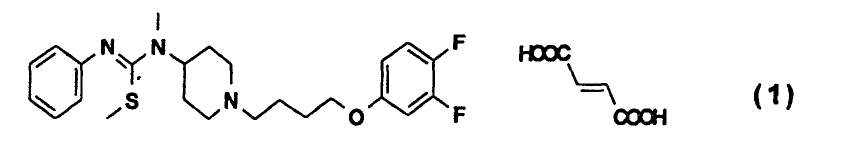 Formule brute : C₂₈H₃₅F₂N₃O₅S
Masse moléculaire : 563,65
Cristaux blancs
Point de fusion : 151-152 °C
**RMN** (DMSO d₆) δ : 1,5-1,8 (m,6H) ; 1,85-1,95 (m, 2H) ; 2 (s, 3H) ; 2,25 (t, 2H) ; 2,45-2,65 (m, 2H) ; 2,9 (s, 3H) ; 3,05-3,15 (m, 2H) ; 3,97 (t, 2H) ; 4,1-4,3 (m, 1 H) ; 6,57 (s, 2H) ; 6,76 (m, 3H) ; 6,9 (t, 1H) ; 7,0-7,12 (m, 1H) ; 7,2 (t, 2H) ; 7,44 (q, 1H), 12-13 (m, 2H).

### Exemple 2 :

### Hydrogénofumarate de la 2-éthyl-1-[1-[4-(3,4-difluorophénoxy)butyl]pipéridin-4-yl]-1-méthyl-3-phényl isothiourée (2).

Une solution de 2 g (4,61 mmol) de 1-méthyl-3-phényl-1-[1-[4-(3,4-difluoro-phénoxy)butyl]pipéridin-4-yl] thiourée (préparée dans l'exemple **1.1**) dans 30 ml d'éthanol est traitée avec 387 µl (754 mg ou 4,84 mmol) d'iodoéthane et agitée une nuit à 25 °C, puis on ajoute de nouveau 199 µl supplémentaires d'iodoéthane et on agite 24 h de plus. Le mélange est évaporé à siccité ; par trituration dans l'éther on élimine un insoluble formé par l'iodohydrate de la thiourée de départ. Le filtrat éthéré est évaporé à siccité, repris dans du chlorure de méthylène et une solution de soude 2N. Les phases sont séparées, la phase aqueuse est reextraite au CH₂Cl₂. La phase organique totale est lavée à l'eau, à l'eau salée, séchée sur sulfate de sodium anhydre, évaporée à siccité et purifiée par flash-chromatographie de la manière habituelle pour donner une huile jaune pâle (m = 1,3 g ; Rdt : 61 %), la base est ensuite salifiée avec l'acide fumarique comme décrit dans l'exemple **1.2**, pour donner 1,31 g (Rdt 48 %) de cristaux de **formule 2**. 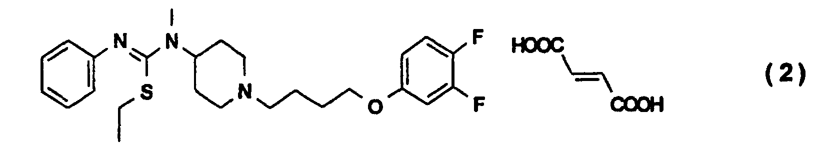 Formule brute : C₂₉H₃₇F₂N₃O₅S
Masse moléculaire : 577,57
Cristaux blancs
Point de fusion : 151-152 °C
**RMN** (DMSO d6) δ : 1,05 (t, 3H) ; 1,5-1,8 (m, 6H) ; 1,80-2,05 (m, 2H) ; 2,23 (t, 2H) ; 2,38 (q, 2H) ; 2,45-2,6 (m, 2H) ; 2,9 (s, 3H) ; 3,05-3,15 (m, 2H) ; 3,97 (t, 2H) ; 4,2-4,4 (m, 1H) ; 6,75 (s, 2H) ; 6,76 (d, 2H) ; 6,90 (t, 1H) ; 7-7,1 (m, 1H), 7,2 (t, 2H) ; 7,33 (q, 1H).

### Exemple 3 :

### Hydrogénofumarate de la 1-éthyl-1-[1-[4-(3,4-difluorophénoxy)butyl]piperidin-4-yl]-2-méthyl-3-phényl isothiourée (3).

**3.1**) Chlorhydrate de la 4-éthylamino-1-[4-(3,4-difluorophénoxy)butyl]pipéridine (**3.1**). Une solution de 6 g de 1-[4-(3,4-difluorophénoxy)butyl]pipéridin-4-one (21,2 mmol) préparée comme décrit dans le brevet WO97.05134 dans un mélange de 70 ml de CH₂Cl₂ et 5 ml de MeOH, est traitée avec 1,73 g (21,2 mmol) de chlorhydrate d'éthylamine. Le mélange est agité 2 h à 25°C puis refroidi sur bain de glace, puis on ajoute 5,39 g (25,4 mmol) de triacétoxyborohydrure de sodium et 1,25 ml d'acide acétique goutte à goutte. Après 1 h d'agitation à 0°C, on laisse revenir à 25 °C et on agite une nuit à cette température. Le mélange réactionnel est versé dans la glace et on alcalinise (avec de la soude 2N) jusqu'à pH = 12. On extrait plusieurs fois au chlorure de méthylène, puis on lave à l'eau et à l'eau salée. Après séchage sur sulfate de sodium anhydre, le sel minéral est éliminé et le filtrat est évaporé à siccité pour donner 6,5 g d'huile brune. Cette huile est reprise dans 40 ml d'éthanol et salifiée avec une solution chlorhydrique éthanolique pour donner 5,8 g (Rdt : 71 %) de composé de **formule 3.1.** 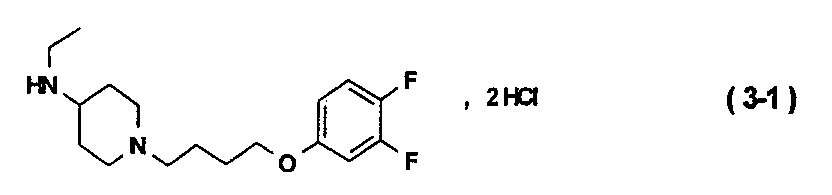 Formule brute : C₁₇H₂₈Cl₂F₂N₂O
Masse moléculaire : 385,33
Cristaux blanc cassé
Point de fusion (déc.) 200 °C
**RMN** : (DMSO d₆) δ : 1,22 (t, 3H); 1,6-2,4 (m, 8H); 2,8-3,7 (m, 9H); 3,99 (t, 2H)); 6,72-6,82 (m, 1H); 6,95-7,15 ( (m, 1H); 7,35 (q, 1H); 9,2-9,7 (m, 2H); 10,5 -11,6 (m, 1H).

**3.2**) 1-éthyl-1-[1-[4-(3,4-difluorophénoxy)butyl]pipéridin-4-yl-]-3-phényl thiourée (**3.2**). En utilisant le mode opératoire décrit dans l'exemple **1.1** mais en partant de 1,4 g (10,4 mmol) de composé **3.1** précédent on prépare avec un rendement de 83 % la thiourée de **formule 3.2. :** 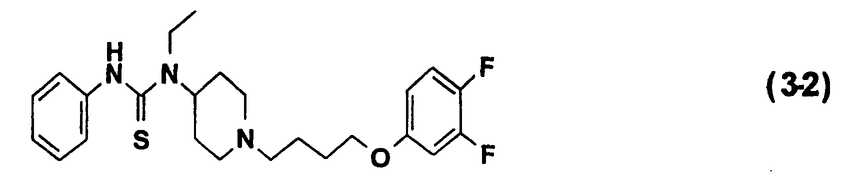 Formule brute : C₂₄H₃₁F₂N₃OS
Masse moléculaire : 447,57
Poudre beige clair
Point de fusion : 133-134 °C
**RMN** (DMSO d₆) δ : 1,15 (t, 3H) ; 1,4-1,8 (m, 8H) ; 1,85-2 (m, 2H) ; 2,31 (t, 2H) ; 3,6 (q, 2H) ; 3,98 (t, 2H) ; 5,03 (m, 1H) ; 6,7-6,8 (m, 1H) ; 7,02-7,18 (m, 2H) ; 7,2- 7,4 (m, 5H) ; 8,89 (s, 1H).

**3.3**) Hydrogénofumarate de la 1-éthyl-1-[1-[4-(3,4-difluorophénoxy)butyl]pipéridin-4-yl]-2-méthyl-3-phényl isosthiourée (**3**).En opérant comme décrit dans l'exemple **1.2** en partant de 2 g de thiourée **3.2** précédente, on prépare avec un rendement de 48 % le composé de **formule 3 :** 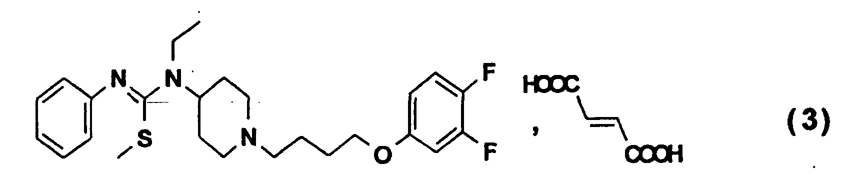 Formule brute : C₂₉H₃₇F₂N₃O₅S
Masse moléculaire : 577,67
Cristaux blancs
Point de fusion : 120 - 121 °C
**RMN** (DMSO d₆) δ : 1,12 (t, 3H) ; 1,5-1,95 (m, 6H) ; 1,90 (s, 3H) ; 2,23 (t, 2H) ; 2,45-2,6 (m, 2H) ; 3,10 (d,d, 2H) ; 3,25-3,6 (m, 2H) ; 3,97 (t, 2H) ; 4,1-4,3 (m, 1H) ; 6,57 (s, 2H) ; 6,7-6,85 (m, 2H) ; 6,89 (t, 1H) ; 7,02-7,1 (m, 1H) ; 7,23 (t, 2H) ; 7,28- 7,4 (t d, 1H).

### Exemple 4 :

### Hydrogénofumarate de la 1-isobutyl-1-[1-[4-(3,4-difluorophénoxy)butyl]pipéridin-4-yl]-2-méthyl-3-phényl isothiourée (4).

**4.1)** Dichlorohydrate de 4-isobutylamino-1-[4-(3,4-difluorophénoxy)butyl]pipéridine (**4.1**). L'amination réductrice réalisée comme décrit dans l'exemple **3.1,** mais en partant de 1,67 ml (1,3 g ou 16,8 mmol) d'isobutyl amine, permet de préparer 5,2 g (Rdt 75 %) de dichlorhydrate de **formule 4.1 :** 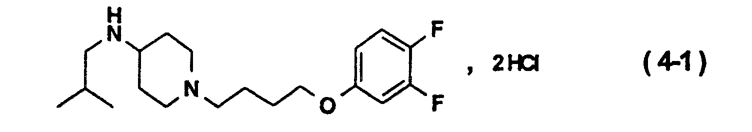 Formule brute : C₁₉H₃₂Cl₂F₂N₂O
Masse moléculaire : 413,38
Cristaux blanc cassé
Point de fusion (déc.) : > 205 °C
**RMN** (DMSO d₆) δ : 0,96 (d, 6H) ; 1,6-1,85 (m, 4H) ; 1,9-2,15 (m, 2H) ; 2,2-2,3 (m, 2H) ; 2,7-3,25 (m, 7H) ; 3,5-3,7 (m, 2H) ; 3,99 (t, 2H) ; 6,6-6,8 (m, 1H) ; 7,03- 7,12 (m, 1H) ; 7,3-7,4 (m, 1H) ; 9-9,3 (m, 2H) ; 10,6-11 (m, 1H).

**4.2)** 1-isobutyl-1-[1-[4-(3,4-difluorophénoxy)butyl] piperidin-4-yl]-3-phényl thiourée (**4.2**). La condensation de 1g(7,4 mmol) d'isothiocyanate de phényle sur 3,06g (7,4 mmol) de composé **4.1** précédent, selon le protocole de l'exemple **1.7**, donne 3,88g (Rdt 91%) de thiourée correspondante de formule **4.2 :** 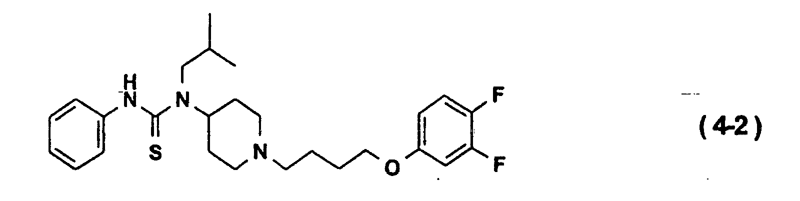 Formule brute : C₂₆H₃₅F₂N₃OS
Masse moléculaire : 475,62
Cristaux blanc cassé
Point de fusion : 132 °C
**RMN** (DMSO d₆) δ : 0,87 (d, 6H) ; 1,45-1,8 (m, 8H) ; 1,9 (t, 2H) ; 2-2,2 (m, 1H) ; 2,3 (t, 2H) ; 2,85-3 (m, 2H) ; 3,2-3,5 (m, 2H) ; 3,98 (t, 2H ; 5 (m, 1H) ; 6,7-6,85 (m, 1H) ; 7-7,15 (m, 2H) ; 7,2-7,48 (m, 5H), 8,98 (s, 1H).

**4.3**) Hydrogénofumarate de la 1-isobutyl-1-[1-[4-(3,4-difluorophénoxy)butyl] piperidin-4-yl]-2-méthyl-3-phényl isothiourée (**4**). La S-méthylation de 1,5 g (3,15 mmol) de la thiourée **4.2** précédente selon le protocole de l'exemple **1.2** donne 620 mg (Rdt : 34 %) d'isothiourée de **formule 4 :** 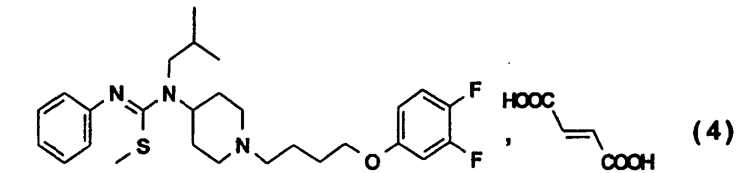 Formule brute : C₃₁H₄₁F₂N₃O₅S
Masse moléculaire : 605,73
Cristaux blancs
Point de fusion : 131 - 132 °C
**RMN** (DMSO d₆) δ : 0,84 (d, 6H), 1,5-2,05 (m, 9H) ; 2,01 (s, 3H) ; 2,134 (t, 2H) ; 2,45-2,6 (m, 2H) ; 3,05-3,15 (m, 4H ; 3,97 (t, 2H) ; 4-4,1 (m, 1H) ; 6,58 (s, 2H) ; 6,7 -6,8 (m, 3H) ; 6,91 (t, 1H) ; 7-7,09 (m, 1H) ; 7,22 (t, 2H) ; 7,28-7,4 (m, 1H) ; 11-13 (m, 2H).

### Exemple 5:

### Hydrogénofumarate de la 1,2-diméthyl-1-[1-[5-(3,4-difluoro-phénoxy)pentyl]pipéridin-4-yl]-3-phényl isothiourée (5) :

**5.1**) 1-[5-(3,4-difluorophénoxy)pentyl]pipéridin-4-one (**5.1**). La condensation de 14 g (50 mmol) de 4-(5-bromopentyloxy)-1,2-difluorobenzène sur 7,13 g (50 mmol) de 1,4-dioxa-8-azaspiro[4,5]décane, selon le procédé d**'Ismaiel A.M.** et col (*J. Med. Chem*. 1993, *36*, 2519-25) donne, après déprotection, 13,6 g (Rdt 91 %) d'une huile orangée de **formule 5.1 :** 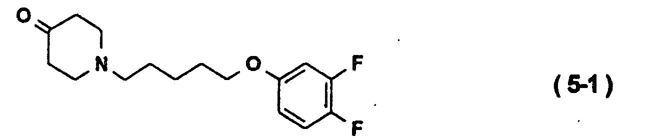 Formule brute : C₁₆H₂₁F₂NO₂
Massse moléculaire : 297,34
**RMN** (CDCl₃) δ : 1,5-1,9 (m, 8H) ; 3-2,8 (m, 8H) ; 3,8-4 (m, 2H) ; 6,5-6,6 (m, 1H) ; 6,65-6,75 (m, 1H) ; 7-7,1 (m, 1H).

**5.2**) Dichlorhydrate de la 1-[5-(3,4-difluorophénoxy) pentyl]-4-méthylamino pipéri- dine **5.2.** En partant de 10 g (33,6 mmol) de cétone **5.1** précédente et de 2,27 g (33,6 mmol) de chlorhydrate de méthylamine et en effectuant l'amination réductrice dans les mêmes conditions que celles de l'exemple **3.1**, on prépare 7,8 g (Rdt: 74 %) de **composé 5.2 :** 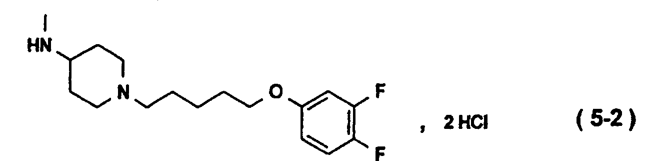 Formule brute : C₁₇H₂₈Cl₂F₂N₂O
Masse moléculaire : 385,33
Cristaux crème
Point de fusion (déc.) : 202 - 205 °C
**RMN** (DMSO, d₆) δ : 1,3-1,45 (m, 2H) ; 1,65-1,8 (m, 4H) ; 1,9-2,05 (m, 2H) ; 2,2- 2,6 (m, 2H) ; 2,51 (d, 3H) ; 2,85-3 (m, 5H) ; 3,5-3,6 (m, 2H) ; 3,97 (t, 2H) ; 6,7-6,8 (m, 1H) ; 7-7,12 (m, 1H) ; 7,34 (t.d, 1H ) ; 9,2-9,7 (m, 2H) ; 10,6- 11,2 (m, 1H).

**5.3**) 1-[1-[5-(3,4-difluorophénoxy)pentyl]pipéridin-4-yl]-1-méthyl-3-phényl thiourée (**5.3**). En appliquant le protocole de l'exemple **1.1** à 1 g (7,4 mmol) de diamine précédente **5.2,** on prépare avec un rendement de 91 % le composé de **formule 5.3 :** 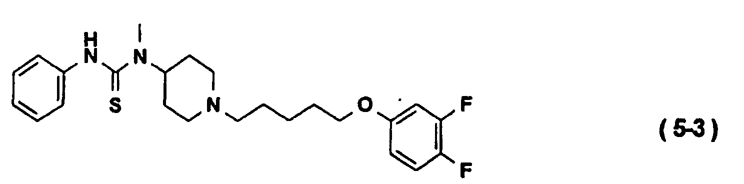 Formule brute : C₂₄H₃₁F₂N₃OS
Masse moléculaire : 447,57
Cristaux crème
Point de fusion : 90 °C
**RMN** (CDCl₃) δ : 1,4-1,6(m, 4H) ; 1,7-1,9 (m, 6H) ; 2,1-2,2 (m, 2H) ; 2,3-2,5 (m, 2H) ; 2,9-3,1 (m, 5H) ; 3,9 (t, 2H) ; 5,2 (m, 1H) ; 6,5-6,6 (m, 1H) ; 6,64-6,75 (m, 1H) ; 7-7,1 (m, 2H) ; 7,15-7,3 (m, 3H) ; 7,3-7,6 (m, 2H).

**5.4)** Hydrogénofumarate de la 1,2-diméthyl-1-[1-[5-(3,4-difluorophénoxy)pentyl]pipéridin-4-yl]-3-phényl isothiourée (**5**). Par réaction de 1,5 g de thiourée précédente avec 230 µl d'iodure de méthyle selon le procédé de l'exemple **1.2** on prépare 600 mg (Rdt: 37 %) de cristaux blancs de **formule 5 :** 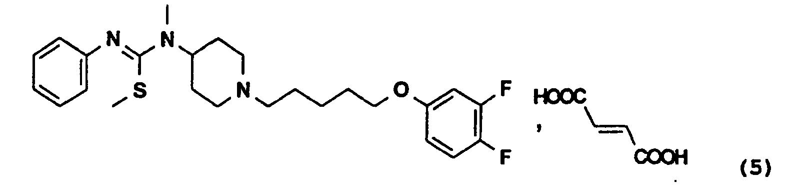 Formule brute : C₂₉H₃₇F₂N₃O₅S
Masse moléculaire : 577,67
Poudre blanche
Point de fusion : 129 - 130 °C
**RMN** (DMSO, d₆)δ: 1,3-1,45 (m, 2H); 1,45-1,6 (m, 2H); 1,65-1,75 (m, 4H); 1,8 -1,91 (m, 2H); 2 (s, 3H); 2,24 (t, 2H); 2,4-2,6 (m, 2H); 2,9 (s, 3H); 3,10 (d, 2H ; 3,95 (t, 2H); 4,1-4,3 (m, 1H); 6,57 (s, 2H); 6,76 (d, 3H) ; 6,9 (t, 1H) ; 7,01-7,09 (m, 1H); 7,21 (t, 2H); 7, 33 (q, 1H).

### Exemple 6 :

### Hydrogénofumarate de la 1,2-diméthyl-1-[1-[3-(3,4-difluorophénoxy)propyl] pipérin-4-yl]-3-phényl isothiourée (6).

**6.1)** 1-[1-[3-(3,4-difluorophénoxy)propyl]piperidin-4-yl]-1-méthyl-3-phényl thiourée (**6.1**). La condensation de 2 g (14,8 mmol) d'isothiocyanate de phényle, sur 5,3 g (14,8 mmol) de dichlorhydrate de 1-[3-(3,4-difluorophénoxy)propyl]-4-méthyl amino- pipéridine (cf. WO 97.05134) selon le procédé de l'exemple **1.1,** donne 5,5 g (Rdt : 88 %) de cristaux de thiourée base de **formule 6.1 :** 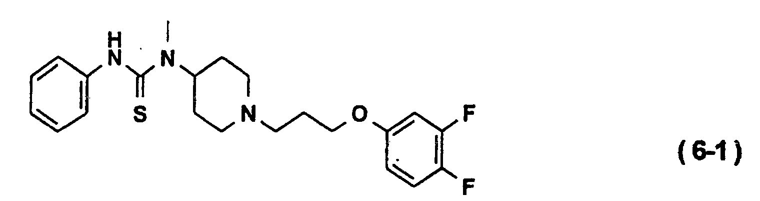 Formule brute : C₂₂H₂₇F₂N₃OS
Masse moléculaire : 419,524
Cristaux blanc cassé
Point de fusion : 114 - 115 °C
**RMN**_(CDCl₃) δ : 1,7-2,03 (m, 6H) ; 2,15 (t, 2H) ; 2,51 (t, 2H) ; 3-3,05 (m, 2H) ; 3,04 (s, 3H) ; 3,96 (t, 2H) ; 5,1-5,25 (m, 1H) ; 6,5-6,6 (m, 1H) ; 6,65-6,78 (m, 1H) ; 7 -7,1 (m, 2H) ; 7,15-7,28 (m, 3H) ; 7,35 (t, 21).

L'hydrogénofumarate de la base précédente **6.1** préparé de la manière habituelle dans l'éthanol fond à 159 °C.

**6.2)** Hydrogénofumarate de la 1,2-diméthyl-1-[1-[3-(3,4-difluorophénoxy)propyl]pipéridin-4-yl]-3-phényl isothiourée (6). La S-méthylation de 1,5 g (3,58 mmol) de la thiourée précédente **6.1** par 245 µl d'iodure de méthyle, selon le protocole de l'exemple **1.2,** donne 615 mg (Rdt : 32 %) de **composé 6** de formule : 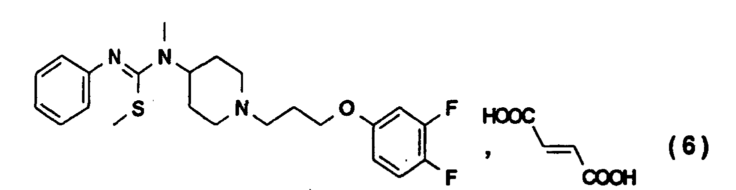 Formule brute : C₂₇H₃₃F₂N₃O₅S
Masse moléculaire : 549,62
Cristaux blancs
Point de fusion : 106 - 107 °C
**RMN** (DMSO d₆) δ : 1,66-1,7 (m, 2H) ; 1,75-1,95 (m, 4H) ; 2 (s, 3H) ; 2,05-2,2 (m, 2H) ; 2,45-2,6 (m, 2H) ; 2,9 (s, 3H) ; 3-3,1 (m, 2H) ; 4 (t, 2H) ; 4,07-4,25 (m, 1H) ; 6,59 (s, 2H) ; 6,76 (d, 3H) ; 6,9 (t, 1H) ; 7,02-7,1 (m, 1H) ; 7,20 (t, 2H) ; 7,33 (q, 1H) ; 12-14 (m, 2H).

### Exemple 7 :

### Hydrogénofumarate de la 1,2-diméthyl-1-[1-[4-(4-fluorophénoxy)butyl]pipéridin-4-yl]-3-phényl isothiourée (7).

**7.1)** 1-méthyl-1-[1-[4-(4-fluorophénoxy)butyl]pipéridin-4-yl]-3-phényl thiourée (**7.1**).

La condensation de 2 g (14,8 mmol) d'isothiocyanate de phényle, sur 5,25 g (14,8 mmol) de dichlorhydrate de 1-[4-(4-fluorophénoxy)butyl]-4-méthylamino pipéridine, (cf. WO 9705134) selon le protocole de l'exemple **1.1,** fournit 5,5 g (Rdt : 90 %) de cristaux blancs de **formule 7.1 :** 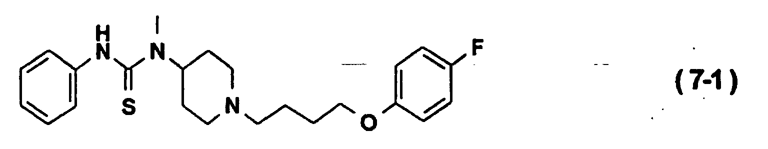 Formule brute : C₂₃H₃₀FN₃OS
Masse moléculaire : 415,55
Cristaux blancs
Point de fusion : 161 - 162 °C
hydrogénofumarate : F = 167 °C)
**RMN** (DMSO d₆) δ: 1;5-1,8 (m, 8H); 1,93 (t, 2H); 2,32 (t, 2H); 2,94 (d, 2H); 3,03 (s, 3H); 3,95 (t, 2H); 5,02 (m, 1H); 5,9-6,97 (m, 2H); 7,05-7,15 (m, 3H); 7,25-7,31) (m, 4H); 8,97 (s, 1H).

**7.2**) Hydrogénofumarate de la 1,2-diméthyl-1-[1-[4-(4-fluorophénoxy)butyl] pipéridin-4-yl]-1-phényl isothiouré (**7**). La S-méthylation de 1,5 g (3,6 mmol) du composé précédent selon le mode opératoire de l'exemple **1.2,** donne 620 mg (Rdt : 32 %) de cristaux blancs de **formule 7 :** 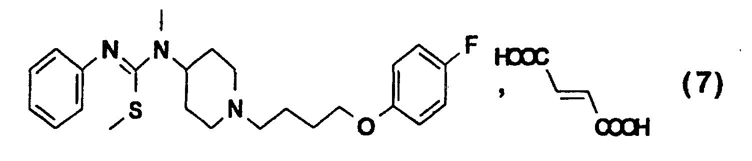 Formule brute : C₂₈H₃₆FN₃O₅S
Masse moléculaire : 545,66
Cristaux blancs
Point de fusion : 133 - 134 °C
**RMN** (DMSO d₆)δ:1,5-1,73 (m, 6H); 1,8-1,93 (m, 2H); 2 (s, 3H); 2,24 (t, 2H) ; 2,4-2,6 (m, 2H); 2,89 (s, 3H); 3,05-3,5 (m, 1H) ; 3,95 (t, 2H); 4,1-4,3 (m, 1H); 6,58 (s, 2H); 6,76 (d, 2H); 6,8-7 (m, 3H); 7,05-7,15 (m, 2H); 7,20 (t, 2H).

### Exemple 8 :

### Hydrogénofumarate de la 1,2-diméthyl-1-[1-[2-(4-fluorophénoxy)éthyl]pipéridin-4-yl]-3-phényl isothiourée (8).

**8.1)**1-méthyl-1-[1-[2-(4-fluorophénoxy)éthyl]pipéridin-4-yl]-3-phényl thiourée **(8.1)**. L'action de 680 mg (5 mmol) d'isothiocyanate de phényle, sur 1,63 g (5 mmol) de dichlorhydrate 1-[2-(4-fluorophénoxy)éthyl]-4-méthylamino pipéridine, selon le protocole de l'exemple **1.2,** donne 1,73 g (Rdt : 89 %) de poudre beige de **formule 8.1 :** 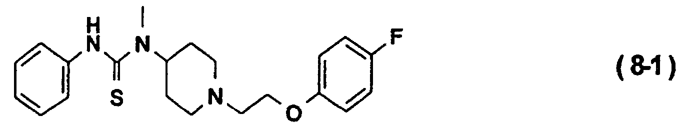 Formule brute : C₂₁H₂₆FN₃OS
Masse moléculaire : 387,50
Poudre beige
Point de fusion : 142 °C
**RMN** (CDCl₃) δ : 1,72-1,9 (m, 4H) ; 2,3 (t.d, 2H) ; 2,81 (t, 2H) ; 3,07 (s, 3H) ; 3,05-3,12 (m, 2H) ; 4,06 (t, 2H) ; 5,1-5,2 (m, 1H) ; 6,84 (d.d, 2H) ; 6,97 (t, 2H) ; 7,04 (s, 1H) ; 7,15-7,27 (m, 3H) ; 7,34 (t, 2H).

**8.2)** Hydrogénofumarate de la 1,2-diméthyl-1-[1-[2-(4-fluorophénoxy)éthyl]pipéridin-4-yl]-3-phényl isothiourée (**8**). En partant de 1,71 g (4,4 mmol) de thiourée précédente **8.1** et en l'alcoylant avec 290 µl d'iodure de méthyle, on prépare 712 mg (Rdt : 31 %) de cristaux blancs de **formule 8 :** 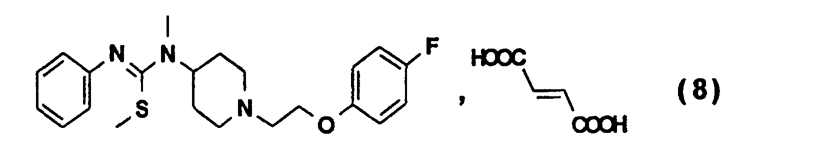 Formule brute : C₂₆H₃₂FN₃O₅S
Masse moléculaire : 517,60
Cristaux blancs
Point de fusion : 120 °C
**RMN** (DMSO d₆) δ : 1,5-1,7 (m, 2H) ; 1,75-1,9 (m, 2H) ; 1.99 (s, 3H) ; 2,20 (t, 2H) ; 2,75 (t, 2H) ; 2,9 (s, 3H) ; 3,07 (d, 2H) ; 4,06 (t, 2H) ; 4,1-4,25 (m, 1H) ; 6,61 (s, 2H) ; 6,76 (d, 2H) ; 6,9 (t, 1H) ; 6,92-6,98 (m, 2H) ; 7,05-7,15 (m, 2H) ; 7,20 (t, 2H) ; 7,20 (t, 2H) ; 12-14 (m, 2H).

### Exemple 9 :

### Hydrogénofumarate de la 1,2-diméthyl-1-[1-[4-[4-méthoxyphénoxy)butyl]pipéridin-4-yl]-3-phényl isothiourée (9).

**9.1)** Dichlorhydrate de la 1-[4-(4-méthoyphénoxy)butyl]-4-méthylamino pipéridine (**9.1**). L'amination rédutrice de 6 g (21,6 mmol) de la 1-[4-(4-méthoxy-phénoxy)butyl]-4-pipéridone (préparé selon **Ismaiel** et coll. *J. Med. Chem*. 1993, *36*, 2519-25) en présence de 1,35 g (20 mmol) de chlorhydrate de méthylamine et de 4,6 g (21,6 mmol) de triacétoxy borohydrure de sodium, selon la description de l'exemple **3.1,** permet de préparer 5,3 g (Rdt : 67 %) de poudre blanche de formule **9.1 :** 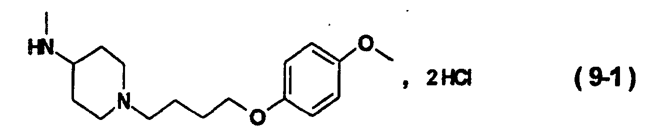 Formule brute : C₁₇H₃₀Cl₂N₂O₂
Masse moléculaire : 365,34
Poudre blanche
**RMN** (DMSO d₆) δ : 1,5-2,3 (m, 8H) ; 2,85-3,25 (m, 5H) ; 3,33 (s, 3H) ; 3,52-3,62 (m, 2H) ; 3,7 (s, 3H) ; 3,92 (d, 2H) ; 6,8-6,9 (m, 4H) ; 9,3-9,6 (m, 2H) ; 10,5-11 (m, 1H).

**9.2)** 1-méthyl-1-[1-[4-(4-méthoxyphénoxy)butyl]pipéridin-4-yl]-3-phényl thiourée (**9.2**). L'addition de 2,5 g (6,84 mmol) de composé **9.1** précédent, sur 925 mg (6,84 mmol) d'isothiocyanate de phényle selon le protocole de l'exemple **1.1**, donne 2,70 g (Rdt : 92 %) de thiourée de formule **9.2 :** 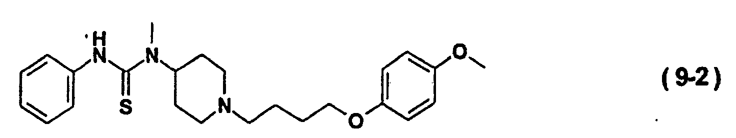 Formule brute : C₂₄H₃₃N₃O₂S
Masse moléculaire : 427,58
Poudre beige
Point de fusion : 111 °C
**RMN** (CDCl₃) δ : 1,65-2,7 (m, 12H) ; 3,06 (s, 3H) ; 3,06-3,25 (m, 2H) ; 3,77 (s, 3H) ; 3,93 (t, 2H) ; 5,3 (m, 1H) ; 6,83 (s, 4H) ; 7,08 (s, 1H) ; 7,15-7,3 (m, 3H) ; 7 ,33 (t, 2H).

**9.3)** Hydrogénomaléate de la 1,2-diméthyl-1-[1-[4-(4-méthoxyphénoxy)butyl] pipéridin-4-yl]-3-phényl isothiourée (**9**). La S-méthylation de 1,5 g (3,5 mmol) de thiourée précédente **9.2,** avec 240 µl d'iodure de méthyle selon **1.2,** conduit à la préparation de 690 mg (Rdt : 35 %) d'isothiourée de **formule 9 :** 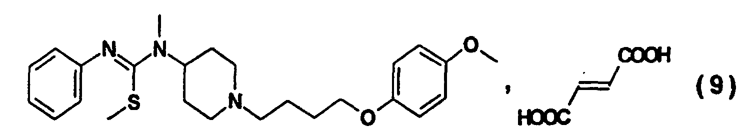 Formule brute : C₂₉H₃₉N₃O₆S
Masse moléculaire : 557,689
Cristaux blancs
Point de fusion : 120 °C
**RMN** (DMSO d₆) δ: 1,5-1,75 (m, 6H); 1,8-1,87 (m, 2H); 2 (s, 3H); 2,35-2,7 (m, 2H); 2,9 (s, 3H) ; 3,09 (d, 2H); 3,69 (s, 3H); 3,91 (t, 2H); 4,12-4,3 (m, 1H); 6,58 (s, 2H); 6,77 (d, 2H); 6,8-6,95 (m, 5H) ; 7,21 (t, 2H).

### Exemple 10 :

### Hydrogénofumarate de la 1,2-diméthyl-1-[1-[4-(3,4-diméthylphénoxy)butyl] pipéridin-4-yl]-3-phényl isothiourée (10).

**10.1**)Dichlorhydrate de la 1-[4-(3,4-diméthylphénoxy)butyl]-4-méthylamino pipéri-dine (**10.1**). Le 1-4-dibromobutane condensé sur le 3,4-diméthylphénol selon **Ismaiel** et coll. (*J. Med. Chem.* 1993, *36*, 2519-25) donne, avec un rendement de 86 %, le 1-[4-bromobutoxy]-3,4-diméthyl benzène. La condensation de ce composé (selon les mêmes auteurs) sur le 1,4-dioxa-8-azaspiro[4,5]décane donne après déprotection la 1-[4-(3,4- diméthylphénoxy)butyl]-4-pipéridone sous forme d'huile orangée avec un rendement de 61 %. L'amination réductrice de cette cétone (4,33 g ou 15,7 mmol) par le chlorhydrate de méthylamine (1,06 g ; 15,7 mmol) en présence de triacétoxy borohydrure de sodium (4,33 g ; 20,5 mmol) selon le protocole de **l'exemple 3.1** permet de préparer 5,95 g (Rdt : 63 %) du composé de **formule 10.1 :** 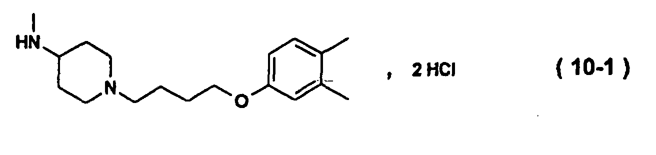 Formule brute : C₁₈H₃₂Cl₂N₂O
Masse moléculaire : 362,37
Poudre blanche
Point de fusion : (décomp) 195-198 °C
**RMN** (DMSO d₆) δ : 1,5-2,1 (m, 6H) ; 2,13 (s, 3H) ; 2,18 (s, 3H) ; 2,18-2,3 (m, 2H) ; 2,8-3,2 (m, 4H) ; 3,33 (m, 3H); 3,4-3,7 (m, 2H); 3,93 (t, 2H) ; 6,6-6,7 (m, 1H) ; 6,73 (s, 1H) ; 7,02 (d, 1H); 9,3-9,7 (m, 2H) ; 10,5-11 (m, 1H).

**10.2)**1-méthyl-1-[1-[4-(3,4-diméthylphénoxy)butyl]piperidin-4-yl]-3-phényl thiourée (**10.2**). L'application du procédé de l'exemple **1.1** à 2,69 g (7,4 mmol) d'amine précédente (**10.1**) et à 1 g (7,40 mmol) d'isothiocyanate de phényle, permet de préparer 2,81 g (Rdt : 89 %) de composé de **formule 10.2 :** 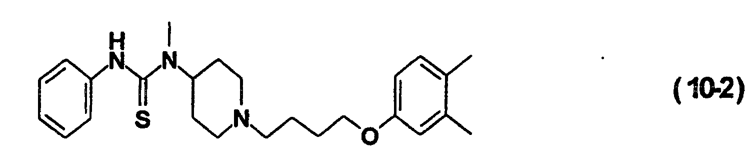 Formule brute : C₂₅H₃₅N₃OS
Masse moléculaire : 425,61
Poudre blanc cassé
Point de fusion : 107 °C
**RMN** (CDCl₃) δ: 1,55-1,95 (m, 8H); 2,1-2,25 (m, 2H); 2,19 (s, 3H); 2,23 (s, 3H); 2,45 (t, 2H); 2,95-3,1 (m, 2H); 3,04 (s, 3H); 3,94 (t, 2H) ; 5,2 (m, 1H) ; 6,63 (dd, 1H) ; 6,7 (d, 1H) ; 7-7,06 (m, 2H) ; 7,1-7,27 (m, 3H) ; 7,36 (t, 2H).

**10.3**) Hydrogénofumarate de la 1,2-diméthyl-1-[1-[4-(3,4-diméthylphénoxy)butyl] pipéridin-4-yl]-3-phényl isothiourée (**10**). Une solution de 1 g (2,35 mmol) de thiourée précédente (**10.2**) dans 10 ml de DMF, est traitée à 25 °C avec 245 µl de diméthyl sulfate, agitée une nuit à 25 °C puis chauffée 1 h à 60 °C. Le mélange est évaporé à siccité, récupéré de la manière habituelle, purifié par flash chromatographie, pour donner 325 mg (Rdt : 32 %) d'une huile jaune clair. Cette huile est reprise dans de l'éthanol et salifiée avec une solution de 80 mg d'acide fumarique dans le même solvant pour donner 340 mg (Rdt : 26 %) du **composé 10** de formule : 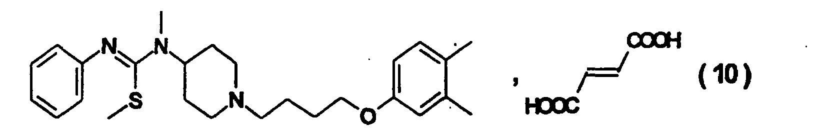 Formule brute : C₃₀H₄₁N₃O₅S
Masse moléculaire : 555,72
Cristaux blancs
Point de fusion : 138-139 °C
**RMN** (DMSO d₆) δ: 1,5-1,7 (m, 6H); 1,75-1,95 (m, 2H) ; 2 (s, 3H) ; 2,1-2,21 (m, 2H) ; 2,12 (s, 3H) ; 2,2 (s, 3H) ; 2,4-2,6 (m, 2H) ; 2,89 (s, 3H) ; 3,02-3,2 (m, 2H) ; 3,92 (t, 2H) ; 4,1-4,3 (m, 1H) ; 6,58 (s, 2H) ; 6,63 (dd, 1H) ; 6,7-6,8 (m, 3H) ; 6,9 (t, 1H) ; 7 (d, 1H) ; 7, 2 (t, 2H).

### Exemple 11 :

### Hydrogénofumarate de la 1,2-diméthyl-1-[1-[4-(2-méthoxy-4-chlorophénoxy) butyl]pipéridin-4-yl]-3-phényl isothiourée (11).

11.1)Dichlorhydrate de la 1-[4-(2-méthoxy-4-chlorophénoxy)butyl]-4-méthylamino pipéridine (11.1). La condensation du 1,4-dibromobutane sur le 2-méthoxy-4-chlorophénol donne, selon **Ismaiel** et coll. le 1-(4-bromobutoxy)-2-méthoxy-4-chlorobenzène avec un rendement de 86 %. L'alcoylation du 1,4-dioxa-8-azaspiro [4,5]décane par ce dérivé fournit, après déprotection, la 1-[4-(2-méthoxy-4-chlorophénoxy)butyl]-4-pipéridone, avec un rendement de 96 %. L'amination réductrice de 4,7 g (∼ 15 mmol) de cétone précédente avec le chlorhydrate de méthylamine (1,02 g) en présence de 4,15 g (19 mmol) de triacétoxy borohydrure de sodium, selon l'exemple **3.1**, donne 3,92 g (Rdt : 65 %) du **composé 11.1** de formule : 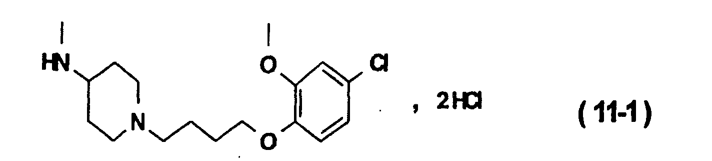 Formule brute : C₁₇H₂₉Cl₃N₂O₂
Masse moléculaire : 399,79
Poudre jaune clair
**RMN** (DMSO d₆) δ : 1,5-2,27 (m, 8H) ; 2,51 (s, 3H) ; 2,8-3,25 (m, 5H) ; 3,4-3,6 (m, 2H) ; 3,8 (s, 3H) ; 3,97 (t, 2H) ; 6,92 (dd, 1H) ; 6,96-6,99 (m, 1H) ; 7,02 (d, 1H) ; 9,3-9,7 (m, 2H) ; 10,2-11,1 (m, 1H).

**11.2)** 1-méthyl-1-[1-[4-(2-méthoxy-4-chlorophénoxy) butyl] pipéridin-4-yl]-3-phényl thiourée (**11.2**). L'application du mode opératoire **1.1** à 2,85 g (7,13 mmol) de diamine précédente **11.1,** conduit à la formation de 2,98 g (Rdt : 90 %) de thiourée de **formule 11.2 :** 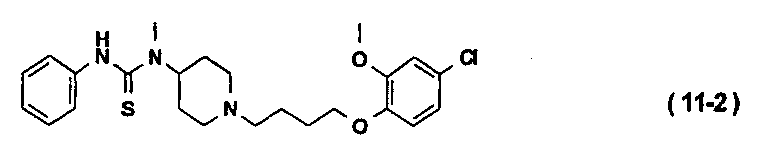 Formule brute : C₂₄H₃₂ClN₃O₂S
Masse moléculaire : 462,04
Cristaux beige clair
Point de fusion : 129 °C
**RMN** (CDCl₃) δ : 1,5-2,1 (m, 8H) ; 2,1-2,3 (m, 2H) ; 2,35-2,6 (m, 2H) ; 3,04 (s, 3H) ; 3-3,2 (m, 2H) ; 3,85 (s, 3H) ; 4,01 (t, 2H) ; 5,24 (m, 1H) ; 6,78 (d, 1H) ; 6,8-6,9 (m, 2H) ; 7,06 (s, 1H) ; 7,1-7,29 (m, 3H) ; 7,35 (t, 2H).

**11.3)** Hydrogénofumarate de la 1,2-diméthyl-1-[1-[4-(2-méthoxy-4-chlorophénoxy) butyl]pipéridin-4-yl]-3-phényl isothiourée (**11**). La S-méthylation de 1,2 g (2,6 mmol) de thiourée précédente **11.2**, selon le procédé de l'exemple **1.2** donne 618 mg (Rdt : 40 %) de dérivé de **formule 11 :** 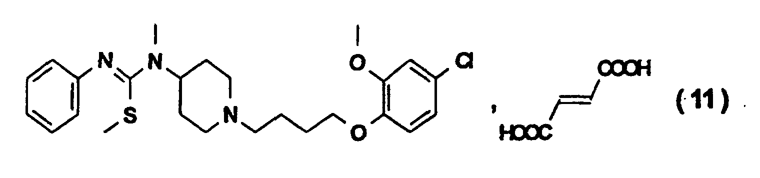 Formule brute : C₂₉H₃₈ClN₃O₆S
Masse moléculaire : 592,13
Cristaux blanc cassé
Point de fusion : 106-107 °C
**RMN** (DMSO d₆) δ: 1,5-1,82 (m, 6H); 1,84-1,97 (m, 2H); 2 (s, 3H), 2,35 (t, 2H); 2,61 (t, 2H); 2,96 (s, 3H); 3,16 (d, 2H); 3,94 (s, 3H); 3,96 (t, 2H); 4,15-4,35 (m, 1H); 6,59 (s, 2H); 6,76 (d, 2H); 6,8-7,1 (m, 4H); 7,21 (t, 2H); 12-14 (m, 2H).

### Exemple 12 :

### Hydrogénofumarate de la 1,2-diméthyl-1-[1-[4-(3,4-difluorophénoxy)butyl]pipéridin-4-yl]-3-(2-fluorophényl) isothiourée (12).

**12.1)** 1-méthyl-1-[1-[4-(3,4-difluorophénoxy) butyl] pipéridin-4-yl]-1-(2-flurophényl) thiourée (**12.1**). L'application du protocole de l'exemple 1.1 à 610 mg (4 mmol) d'isothiocyanate de 2-fluorophényle et 1,20 g (4 mmol) de 1-[4-(3,4-difluorophénoxy)butyl]-4-méthylamino pipéridine donne 1,65 g (Rdt : 91 %) de cristaux blancs de **formule 12.1 :** 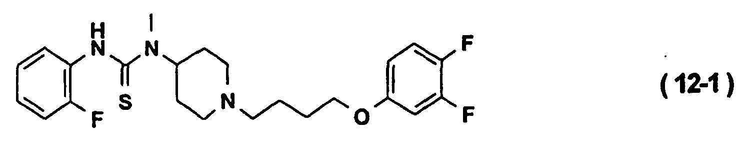 Formule brute : C₂₃H₂₈F₃N₃OS
Masse moléculaire : 451,54
Cristaux blancs
Point de fusion : 126 °C
**RMN** (CDCl₃) δ :1,5-1,95 (m, 8H) ; 2,13 (t, 2H) ; 2,42 (t, 2H) ; 3-3,06 (m, 2H) ; 3,11 (s, 3H) ; 3,94 (t, 2H) ; 5,21 (m, 1H) ; 6,5-6,6 (m, 1H) ; 6,5-6,75 (m, 1H) ; 6,92 (s, 1H) ; 6,8-7,3 (m, 4H) ; 7,75-8,01 (m, 1H).

**12.2)**Hydrogénofumarate de la 1,2-diméthyl-1-[1-[4-(3,4-difluorophénoxy)butyl] pipéridin-4-yl]-3-(2-fluorophényl) isothiourée (**12**). A une solution de 1,60 g (3,54 mmol) de thiourée **12.1** précédente, dans 20 ml de chloroforme, on ajoute 200 mg (3,54 mmol) de CaO et 242 µl (3,9 mmol) d'iodure de méthyle, puis on porte le mélange au reflux pendant 3 heures. Le mélange est filtré puis on lave à l'eau, à l'eau salée et sèche sur sulfate de sodium anhydre. Après élimination du sel minéral le filtrat est évaporé à siccité et purifié par flash-chromatographie en éluant avec un mélange CHCl₃/MeOH/NH₄OH-95/4,5/0,5. On récupère 1,1 g d'huile qui est salifiée de la manière habituelle, pour donner 790 mg (Rdt : 39 %) de composé de **formule 12 :** 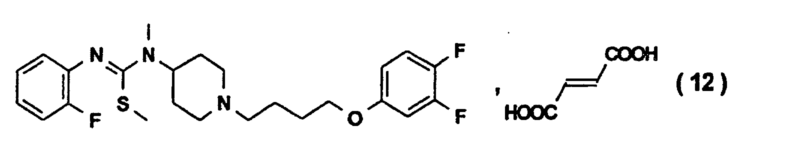 Formule brute : C₂₈H₃₄F₃N₃O₅S
Masse moléculaire : 581,64
Cristaux blancs
Point de fusion : 148 °C
**RMN** (DMSO d₆) δ : 1,5-1,75 (m, 6H); 1,78-1,9 (m, 2H); 2,08 (s, 3H) ; 2,15 (t, 2H); 2,41-2,6 (m, 2H) ; 2,93 (s, 3H); 3,01-3,09 (m, 2H) ; 3,97 (t, 2H) ; 4,13-4,25 (m, 1H); 6,58 (s, 2H); 6,72-6,79 (m, 1H); 6,81-6,87 (m, 1H); 6,9-6,95 (m, 1H); 7,0-7,1 (m, 3H); 7,33 (q, 1H) ; 12-14 (m, 2H).

### Exemple 13 :

### Hydrogénofumarate de la 1,2-diméthyl-1-[1-[4-(3,4-difluorophénoxy)butyl]pipéridin-4-yl]-3-(4-fluorophényl) isothiourée (13).

**13.1)** 1-méthyl-1-[1-[4-difluorophénoxy)butyl]pipéridin-4-yl]-3-(4-fluorophényl) thio- urée (**13.1**). La condensation de 1,2 g (4 mmol) de 1-[4-(3,4-difluorophénoxy) butyl]-4-méthylamino pipéridine, sur 0,61 g (4 mmol) d'isothiocyanate de 4-fluorophényle dans le THF, selon le protocole de l'exemple **1.1** donne 1,75 g (Rdt : 95 %) de poudre de **formule 13.1 :** 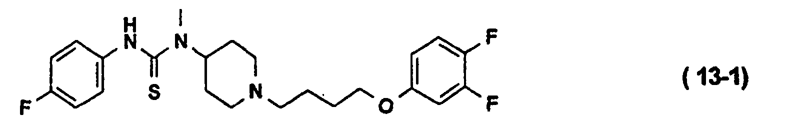 Formule brute : C₂₃H₂₈F₃N₃OS
Masse moléculaire : 451,54
Poudre blanche
Point de fusion : 133 °C
**RMN** (CDCl₃) δ : 1,5-1,9 (m, 8H) ; 2,13 (t, 2H) ; 2,42 (t, 2H) ; 3-3,1 (m, 2H) ; 3,06 (s, 3H) ; 3,92 (t, 2H) ; 5,23 (m, 1H) ; 6,5-6,6 (m, 1H) ; 6,65-6,75 (m, 1H) ; 6,94 (s, 1H) ; 7-7,08 (m, 3H) ; 7,2-7,3 (m, 2H).

**13.2)** Hydrogénofumarate de la 1,2-diméthyl-1-[1-[4-(3,4-difluorophénoxy)butyl] pipéridin-4-yl]-3-(4-fluorophényl) isothiourée (**13**). Par S-méthylation de 1,2 g (2,65 mmol) de thiourée **13.1** précédente, on prépare selon l'exemple **1.2** l'isothiourée **13** avec un rendement de 21 %. 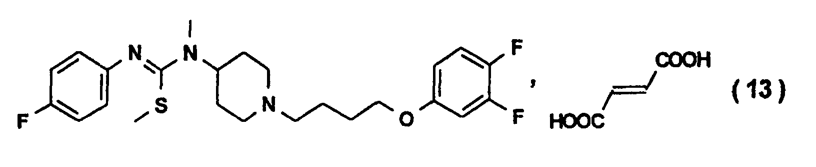 Formule brute :C₂₈H₃₄F₃N₃O₅S
Masse moléculaire : 581,64
Cristaux blancs
Point de fusion : 125 °C
**RMN** (DMSO d₆) δ : 1,5-1,73 (m, 6H) ; 1,74-1,9 (m, 2H) ; 2,02 (s, 3H) ; 2,15 (t, 2H) ; 2,4-2,6 (m, 2H) ; 2,89 (s, 3H) ; 3-3,1 (m, 2H) ; 3,97 (t, 2H) ; 4,1-4,25 (m, 1H) ; 6,5 (s, 2H) ; 6,7-6,8 (m, 3H) ; 6,95-7,1 (m, 3H) ; 7,25-7,4 (m, 1H) ; 11-13 (m, 2H).

### Exemple 14 :

### Hydrogénofumarate de la 1,2-diméthyl-1-[1-[4-(3,4-difluorophénoxy)butyl]pipéridin-4-yl]-3-(4-chlorophényl) isothiourée (14).

**14.1**)1-méthyl-1-[1-[4-(3,4-difluorophénoxy)butyl]pipéridin-4-yl]-3-(4-chlorophényl) thiouré (**14.1**). Selon le procédé de l'exemple 1.1, en partant de 0,85 g (5 mmol) d'isothiocyanate de 4-chlorophényle et de 1,5 g (5 mmol) de 1-[4-(3,4-difluorophénoxy)butyl]-4-méthylamino pipéridine dans 30 ml de THF, on prépare 2,16 g (Rdt : 92 %) de **composé 14.1** de formule : 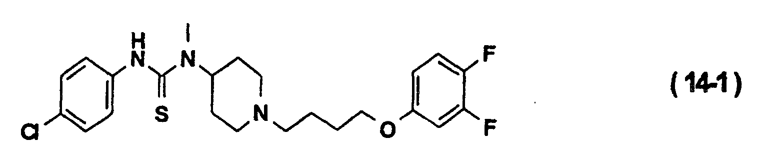 Formule brute : C₂₃H₂₈ClF₂N₃OS
Masse moléculaire : 467,99
Cristaux blanc cassé
Point de fusion : 149 °C
(Point de fusion de l'hydrogénofumarate : 166 °C)
**RMN** (CDCl₃) δ : 1,5-2 (m, 8H) ; 2,14 (t, 2H) ; 2,43 (t, 2H) ; 3-3,1 (m, 2H) ; 3,06 (s, 3H) ; 3,92 (t, 2H) ; 5,12 (m, 1H) ; 6,5-6,6 (m, 1H) ; 6,62-6,75 (m, 1H) ; 6,95 (s, 1H) ; 7-7,08 (m, 1H) ; 7,22 (d, 2H) ; 7,31 (d, 2H).

**14.2)**Hydrogénofumarate de la 1,2-diméthyl-1-[1-[4-(3,4-difluorophénoxy)butyl] pipéridin-4-yl]-3-(4-chlorophényl) isothiourée (**14**). En utilisant le procédé de l'exemple **1.2** en chauffant à 40 °C pendant 2h, un mélange de 1,65 g (3,5 mmol) de thiourée **14.1** précédente et 240 µl d'iodure de méthyle (3,85 mmol) dans 30 ml d'éthanol, on prépare avec un rendement de 36 % le composé **14** de formule : 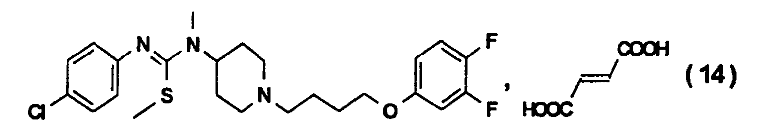 Formule brute : C₂₈H₃₄ClF₂N₃O₅S
Masse moléculaire : 598,09
Cristaux pulvérulents blancs
Point de fusion : 130 °C
**RMN** (DMSO d₆) δ : 1,5-1,75 (m, 6H) ; 1,77-1,9 (m, 2H) ; 2,03 (s, 3H) ; 2,18 (t, 2H) ; 2,4-2,6 (m, 2H) ; 2,9 (s, 3H) ; 3,07 (d, 2H) ; 3,97 (t, 2H) ; 4,1-4,2 (m, 1H) ; 6,58 (s, 2H) ; 6,7-6,8 (m, 3H) ; 7-7,09 (m, 1H) ; 7,2-7,3 (m, 2H) ; 7,3-7,4 (m, 1H).

### Exemple 15 :

### Hydrogénofumarate de la 1,2-diméthyl-1-[1-[4-(3,4-difluorophénoxy)butyl]pipéridin-4-yl]-3-(4-méthylphényl) isothiourée (15).

**15.1)**1-méthyl-1-[1-[4-(3,4-difluorophénoxy)butyl]pipéridin-4yl]-3-(4-méthylphényl) thiourée (**15.1**). Le remplacement de l'isocyanate de phényle par l'isocyanate de para tolyle (0,60 g ; 4 mmol) dans le mode opératoire de l'exemple **1.1** avec le THF comme solvant, conduit avec un rendement de 95 % à la thiourée **15.1** de formule : 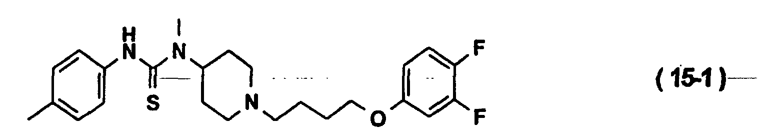 Formule brute : C₂₄H₃₁F₂N₃OS
Masse moléculaire : 447,57
Cristaux blanc cassé
Point de fusion :151 °C
**RMN** (CDCl₃) δ : 1,5-1,95 (m, 8H) ; 2,13 (t, 2H) ; 2,33 (s, 3H) ; 2,42 (t, 2H) ; 2,95-3,1 (m, 5H) ; 3,92 (t, 2H) ; 5,21 (m, 1H) ; 6,5-6,6 (m, 1H) ; 6,65-6,8 (m, 1H) ; 6,98 (s, 1H) ; 7-7,1 (m, 1H) ; 7,1-7,17 (m, 4H).

**15.2)** Hydrogénofumarate de la 1,2-diméthyl-1-[1-[4-(3,4-difluorophénoxy)butyl]pi-péridin-4-yl]-3-(4-méthylphényl) isothiourée (**15**). Par S-méthylation de 1,12 g (2,5 mmol) de thiourée **15.1** précédente, selon le procédé de l'exemple **1.2,** on prépare 830 mg (Rdt : 57 %) de cristaux de formule **15** : 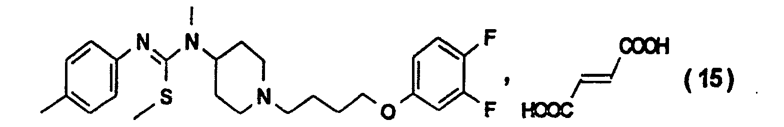 Formule brute : C₂₉H₃₇F₂N₃O₅S
Masse moléculaire : 577,67
Cristaux blancs
Point de fusion : 141 °C
**RMN** (DMSO d₆) δ : 1,5-1,75 (m, 6H) ; 1,8-1,9 (m, 2H) ; 2 (s, 3H) ; 2,18 (t, 2H) ; 2,23 (s, 3H) ; 2,42-2,55 (m, 2H) ; 2,87 (s, 3H) ; 3,07 (d, 2H) ; 3,97 (t, 2H) ; 4,1-4,25 (m, 1H) ; 6,58 (s, 2H) ; 6,64 (d, 2H) ; 6,7-6,8 (m, 1H) ; 7,01 (d, 2H) ; 7,02-7,09 (m, 1H) ; 7,33 (q, 1H).

### Exemple 16 :

### Hydrogénofumarate de la 1,2-diméthyl-1-[1-[4-(3,4-difluorophénoxy)butyl]pipéridin-4-yl]-3-(4-méthoxyphényl) isothiourée (16).

**16.1)** 1-méthyl-1-[1-[4-(3,4-difluorophénoxy)butyl]pipéridin -4-yl]-3-(4-méthoxyphé- nyl) thiourée (**16.1**). En utilisant le protocole de l'exemple **1.1** avec l'isothiocyanate de 4-méthoxyphényle (660 mg ; 4 mmol) comme agent acylant dans le THF on prépare 1,66 g (Rdt : 90 %) de **composé 16.1** de formule : 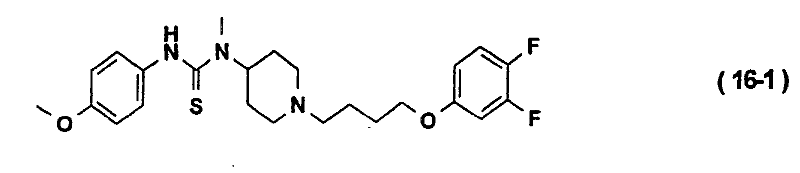 Forme brute : C₂₄F₃₁F₂N₃O₂S
Masse moléculaire : 463,57
Poudre beige
Point de fusion : 122 °C
**RMN** (CDCl₃) δ : 1,5-1,9 (m, 8H) ; 2,14 (t, 2H) ; 2,42 (t, 2H) ; 2,95-3,12 (m, 2H) ; 3,05 (s, 3H) ; 3,81 (s, 3H) ; 3,92 (t, 2H) ; 5,24 (m, 1H) ; 6,5-6,6 (m, 1H) ; 6,65-6,75 (m, 1H) ; 6,88 (d, 2H) ; 6,94 (s, 1H) ; 7-7,08 (m, 1H) ; 7,17 (d, 2H).

**16.2)**Hydrogénofumarate de la 1,2-diméthyl-1-[1-[4-(3,4-difluorophénoxy)butyl] pipéridin-4-yl]-3-(4-méthoxyphényl) isothiourée (**16**). La S-alcoylation réalisée sur 1,15 g (2,5 mmol) de thiourée **16.1** selon le procédé de l'exemple **1.2** donne 692 mg (Rdt : 46 %) d'isothiourée de **formule 16 :** 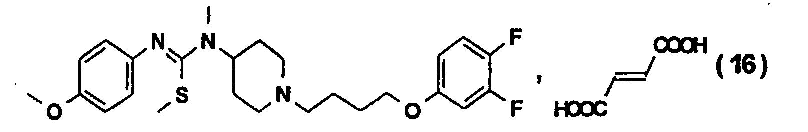 Formule brute : C₂₉H₃₇F₂N₃O₆S
Masse moléculaire : 593,67
Cristaux blancs
Point de fusion : 130 °C
**RMN** (DMSO d₆) δ : 1,4-1,9 (m, 8H) ; 2 (s, 3H) ; 2, 18 (t, 2H) ; 2,4-2,6 (m, 2H) ; 2,87 (s, 3H) ; 3,07 (d, 2H) ; 3,7 (s, 3H) ; 3,97 (t, 2H) ; 4,1-4,25 (m, 1H) ; 6,58 (s, 2H) ; 6,69 (d, 2H) ; 6,72-6,85 (m, 3H) ; 7-7,1 (m, 1H) ; 7,33 (q, 1H).

### Exemple 17 :

### Hydrogénofumarate de la 1,2-diméthyl-1-[1-[4-(3,4-difluorophénoxy)butyl]pipéridin-4-yl]-3-(2,6-diméthylphényl) isothiourée (17).

**17.1)** 1-méthyl-1-[1-[4-(3,4-difluorophénoxy)butyl]pipéridin - 4-yl]-3-(2,6-diméthyl-phényl] thiourée (**17.1**). L'utilisation de 820 mg (5 mmoles) d'isothiocyanate de 2,6- diméthylphényle dans le protocole de l'exemple **1.1** conduit à la formation de 1,96 g (Rdt : 86 %) de thiourée de **formule 17.1 :** 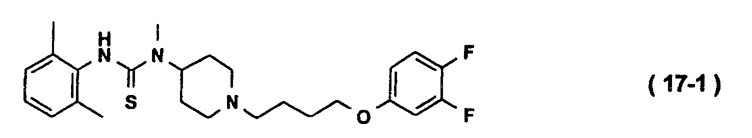 Formule brute : C₂₅H₃₃F₂N₃OS
Masse moléculaire : 461,60
Poudre blanc cassé
Point de fusion : 114 °C
**RMN** (CDCl₃) δ : 1,5-1,9 (m, 8H) ; 2,07-2,18 (m, 2H) ; 2,24 (s, 6H) ; 2, 32-2,5 (m, 2H) ; 3-3,05 (m, 2H) ; 3,09 (s, 3H) ; 3,92 (t, 2H) ; 5,24 (m, 1H) ; 6,5-6,63 (m, 2H) ; 6,66-6,74 (m, 1H) ; 6,95-7,2 (m, 4H).

**17.2)**Hydrogénofumarate de la 1,2-diméthyl-1-[1-[4-(3,4-difluorophénoxy)butyl] pipéridin-4-yl]-3-(2,6-diméthyl-phényl) isothiourée (**17**). La thiourée **17.1** précédente (1,33 g ; 2,88 mmol) est traitée selon le procédé **1.2,** pour donner 720 mg (Rdt : 42 %) d'isothiourée de **formule 17 :** 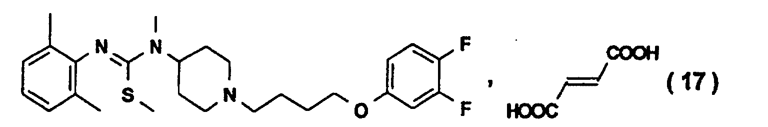 Formule brute : C₃₀H₃₉F₂N₃O₅S
Masse moléculaire : 591,70
Cristaux blanc cassé
Point de fusion : 129 °C
**RMN** (DMSO d₆) δ : 1,5-1,8 (m, 6H) ; 1,8-1,92 (m, 2H) ; 1,97 (s, 6H) ; 2,11 (s, 3H) ; 2,19 (t, 2H) ; 2,4-2,55 (m, 2H) ; 2,87 (s, 3H) ; 3,05-3,13 (m, 2H) ; 3,9-4,08 (m, 3H) ; 6,58 (s, 2H) ; 6,7-6,8 (m, 2H) ; 6,94 (d, 2H) ; 7-7,1 (m, 1H) ; 7,33 (q, 1H).

### Exemple 18 :

### Hydrogénofumarate de la 1,2-diméthyl-1-[1-[4-(3,4-difluorophénoxy)butyl]pipéridin-4-yl]-3-[2,6-diisopropylphényl] isothiourée (18).

**18.1)**1-méthyl-1-[1-[4-(3,4-difluorophénoxy)butyl]pipéridin -4-yl]-3-[2,6-diisopropyl phényl] thiourée **(18.1).** A partir de 1,10 g (5 mmol) d'isothiocyanate de 2,6-di isopropyl phényle dans 20 ml de THF selon le protocole **1.1,** on prépare 2,31 g (Rdt : 89 %) de thiourée de **formule 18.1 :** 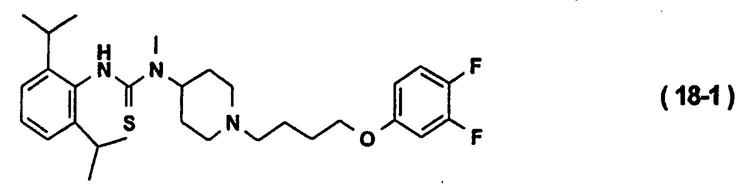 Formule brute : C₂₉H₄₁F₂N₃OS
Masse moléculaire : 517,70
Poudre blanc cassé
Point de fusion : 130 °C ; (Hydrogénofumarate: F = 164 °C)
**RMN** (CDCl₃) δ: 1,16 (d, 6H) ; 1,29 (d, 6H) ; 1,45-1,95 (m, 8H); 2-2,25 (m, 2H) ; 2,3-2,5 (m, 2H) ; 3,03 (t, 2H) ; 3,14 (s, 3H) ; 3,93 (t, 2H) ; 6,47-6,62 (m, 2H) ; 6,65-6,75 (m, 1H) ; 7,05 (q, 1H) ; 7,19 (d, 2H) ; 7,26-7,4 (m, 1H).

**18.2)** 1,2-diméthyl-1-[1-[4-(3,4-difluorophénoxy)butyl] pipéridin-4-yl]-3-[2,6-diiso- propylphényl] isothiourée (**18**). Par S-méthylation de 1,50 g (2,9 mmol) de la thiourée **18.1** précédente, en chauffant 4 h à 50° selon l'exemple **14.2,** on prépare 698 mg (Rdt : 37 %) de S-méthyl isothiourée **18** correspondante : 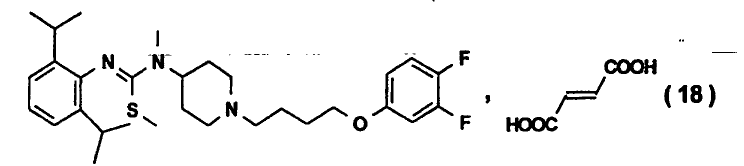 Formule brute : C₃₄H₄₇F₂N₃O₅S
Masse moléculaire : 642,82
Poudre blanc cassé
Point de fusion 194 °C
**RMN** (DMSO d₆) δ : 1,05 (d, 6H) ; 1,13 (d, 6H) ; 1,6-1,85 (m, 6H) ; 1,9-2,1 (m, 2H) ; 2,18 (s, 3H) ; 2,7-3 (m, 4H) ; 2,89 (s, 3H) ; 3,1-3,7 (m, 4H) ; 4 (t, 2H) ; 4,04-4,3 (m, 1H) ; 6,62 (s, 2H) ; 6,7-6,8 (m, 1H) ; 6,91 (t, 1H) ; 6,96 -7,12 (m, 3H) ; 7,35 (q, 1H) ; 11-13 (m, 2H).

### Exemple 19 :

### Hydrogénofumarate de la 1,2-diméthyl-1-[1-[4-(3,4-difluorophénoxy)butyl]pipéridin-4-yl]-3-(3-fluorophényl) isothiourée (19).

**19.1)** 1-méthyl-1-[1-[4-(3,4-difluorophénoxy)butyl]pipéridin -4-yl]-3-(3-fluorophényl) thiourée (**19.1**). L'application du protocole de l'exemple **1.1** à 0,61 g (4 mmol) d'isothiocyanate de 3-fluorophényle permet de préparer 1,87 g (Rdt : 94 %) de poudre blanche de **formule 19.1 :** 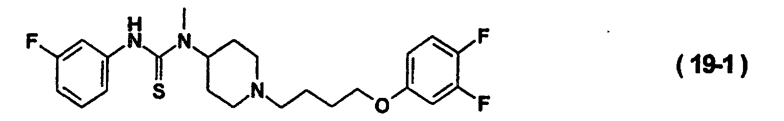 Formule brute : C₂₃H₂₈F₃N₃OS
Masse moléculaire : 451,84
Poudre blanche
Point de fusion : 128 °C
**RMN** (CDCl3) δ : 1,5-1,9 (m, 8H) ; 2,13 (s, 2H) ; 2,35-2,48 (m, 2H) ; 2,95-3 ; (m, 2H) ; 3,04 (s, 3H) ; 3,92 (t, 2H) ; 5,16 (m, 1H) ; 6,5-6,6 (m, 1H) ; 6,65-6,72 (m, 1H) ; 6,88 (m, 1H) ; 6,95-7,1 (m, 4H) ; 7,23-7,4 (m, 1H).

**19.2)** Hydrogénofumarate de la 1,2-diméthyl-1-[1-[4-(3,4-difluorophénoxy)butyl] pipéridin-4-yl]-3-(3-fluorophénoxy) isothiourée (**19**). La S-méthylation de 1,60 g (3,54 mmol) de thiourée **19.1** donne 880 mg (Rdt : 42 %) de composé de **formule 19 :** 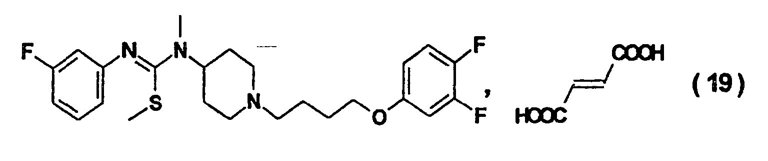 Formule brute : C₂₈H₃₄F₃N₃O₆S
Masse moléculaire : 581,64
Cristaux blancs
Point de fusion : 133 °C
**RMN** (DMSO d₆) δ : 1,5-1,9 (m, 8H) ; 2,03 (s, 3H) ; 2,14 (t, 2H) ; 2,46 (t, 2H) ; 2,9 (s, 3H) ; 3,05 (d, 2H) ; 3,97 (t, 2H) ; 4,12-4,25 (m, 1H) ; 6,5-6,63 (m, 4H) ; 6,67-6,8 (m, 2H) ; 7-7,1 (m, 1H) ; 7,23 (q, 1H) ; 7,33 (q, 1H).

### Exemple 20 :

### Hydrogénofumarate de la 1,2-diméthyl-1-[1-[4-(3,4-difluorophénoxy)butyl]pipéridin-4-yl]-3-(4-nitrophényl) isothiourée (20).

**20.1)** 1-méthyl-1-[1-[4-(3,4-difluorophénoxy)butyl]pipéridin -4-yl]-3-(4-nitrophényl) thiourée (**20.1**). Par condensation de 0,72 g (4 mmol) d'isothiocyanate de 4-nitro- phényle selon l'exemple **1.1**, on prépare 1,71 g (Rdt : 88 %) de poudre jaune de **formule 20.1 :** 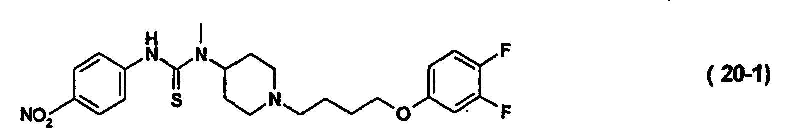 Formule brute : C₂₃H₂₈F₂N₄O₃S
Masse moléculaire : 478,55
Cristaux jaunes pulvérulents
Point de fusion : 108 °C
**RMN** (CDCl₃) δ : 1,5-1,95 (m, 8H) ; 2,14 (t, 2H) ; 2,35-2,5 (m, 2H) ; 3-3,15 (m, 2H) ; 3,11 (s, 3H ) ; 3,93 (t, 2H) ; 5,15 (m, 1H) ; 6,52-6,62 (m, 1H) ; 6,65-6,78 (m, 1H) ; 7,05 (q, 1H) ; 7,16 (s, 1H) ; 7,45 (d, 2H) ; 8,2 (d, 2H).

**20.2)**Hydrogénofumarate de la 1,2-diméthyl-1-[1-[4-(3,4-difluorophénoxy)butyl] pipéridin-4-yl-]-3-(4-nitrophényl) isothiourée (**20**). La S-méthylation de 1,60 g (3,34 mmol) de thiourée précédente **20.1,** selon l'exemple **12.2,** permet de préparer 610 mg (Rdt : 30 %) d'isothiourée de **formule 20 :** 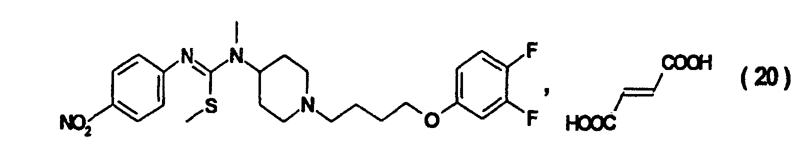 Formule brute : C₂₈H₃₄F₂N₄O₇S
Masse moléculaire : 608,64
Cristaux jaunes
Point de fusion : 132 °C
**RMN** (DMSO d₆) δ : 1,5-1,74 (m, 6H) ; 1,75-1,90 (m, 2H) ; 2,03 (s, 3H) ; 2,1 (t, 2H) ; 2,46 (t, 2H) ; 3,05 (s, 3H) ; 3,1 (d, 2H) ; 3,97 (t, 2H) ; 4,2-4,3 (m, 1H) ; 6,59 (s, 2H) ; 6,74-6,78 (m, 1H) ; 6,95 (d, 2H) ; 7-7,1 (m, 1H) ; 7,33 (q, 1H) ; 8 ,09 (d, 2H) ; 12-14 (m, 2H).

### Exemple 21 :

### Hydrogénofumarate de la 1,2-diméthyl-1-[1-[4-(3,4-difluorophénoxy)butyl]pipéridin-4-yl]-3-(2,6-difluorophényl) isothiourée (21).

**21.1)** 1-méthyl-1-[1-[4-(3,4-difluorophénoxy)butyl]pipéridin -4-yl]-3-(2,6-difluoro-phényl) thiourée (**21.1**). La réaction de 1 g (5,84 mmol) d'isothiocyanate de 2,6-difluorophényle selon la description de l'exemple **1.1,** donne 2,88 g (Rdt : 81 %) de poudre blanche de **formule 21.1 :** 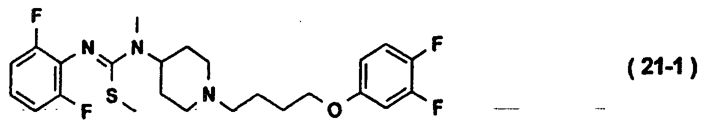 Formule brute : C₂₃H₂₇F₄N₃OS
Masse moléculaire : 469,53
Cristaux blancs pulvérulents
Point de fusion : 120 °C
**RMN** (CDCl₃) 8 : 1,5-2 (m, 8H) ; 2,15 (t, 2H) ; 2,43 (t, 2H) ; 3-3,1 (m, 2H) ; 3,15 (s, 3H) ; 3, 92 (t, 2H) ; 5,19 (m, 1H) ; 6, 39 (s, 1H) ; 6,5-6 ,6 (m, 1H) ; 6,65-6,75 (m, 1H) ; 6,97 (t, 2H) ; 7,05 (q, 1H) ; 7,2-7,6 (m, 1H).

**21.2)**Hydrogénofumarate de la 1,2-diméthyl-1-[1-[4-(3,4-difluorophénoxy)butyl]pipéridin-4-yl]-3-(2,6-difluoro-phényl) isothiourée (**21**). L'action de 0,60 g (4,2 mmol) d'iodure de méthyle sur 1,88 g (4 mmol) de thiourée précédente **21.1** dans 10 ml de DMF selon le protocole **1.2,** permet de préparer 690 mg (Rdt : 29 %) d'isothiourée **21** de formule : 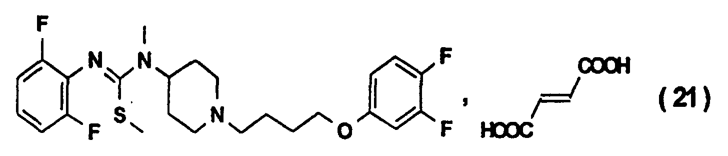 Formule brute : C₂₈H₃₃F₄N₃O₅S
Masse moléculaire :
Cristaux blancs
**RMN** (DMSO d₆) δ : 1,6-2,1 (m, 8H) ; 2,17 (s, 3H) ; 2,7-2,95 (m, 4H) ; 3 (s, 3H) ; 3,35-3,45 (m, 2H) ; 3,99 (t, 2H) ; 4,3-4,5 (m, 1H) ; 6,62 (s, 2H) ; 6,7-6,8 (m, 1H) ; 6,9-7,12 (m, 4H) ; 7,35 (q, 1H) ; 11-13 (m, 2H).

### Exemple 22 :

### Hydrogénofumarate de la 1,2-diméthyl-1-[1-[3-[2-(4-fluorophényl)éthoxy]propyl] pipéridin-4-yl]-3-phényl isothiourée (22) :

**22.1)** 4-fluoro-1-[2-(3-chloropropoxy)éthyl]benzène **(22.1).** Une solution de 86 g (2,14 mol) de NaOH en pastille dans 86 g d'eau est agitée, refroidie à 25 °C puis traitée avec 20 g (0,143 mol) d'alcool 4-fluorophénéthylique ; 113 ml (1,14 mol) de 1-bromo-3-chloropropane, puis avec 4,85 g (14 mmol) d'hydrogénosulfate de tétrabutyl ammonium. La forte agitation est maintenue pendant 4 h à 25 °C puis on extrait à l'éther, lave à l'eau, à l'eau salée et sèche sur sulfate de sodium anhydre. Après élimination du sel minéral, le filtrat est évaporé à siccité et l'huile résiduelle est rectifiée sous vide pour donner 20,3 g (Rdt : 65 %) de produit de formule **22.1 :** 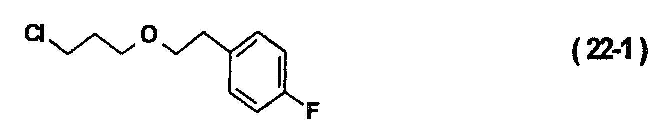 Formule brute : C₁₁H₁₄ClFO
Masse moléculaire : 216,67
Huile incolore
Point d'ébullition : 94-97 °C / 0,4 mbar.
**RMN** (CDCl₃) δ : 2,08 (q, 2H) ; 2,85 (t, 2H) ; 3,46 (t, 2H) ; 3,55 (t, 2H) ; 3,63 (t, 2H) ; 6,97 (t, 2H) ; 7,1-7,2 (m, 2H).

**22.2)**1-[3-[2-(4-fluorophényl)éthoxy]propyl]-4-pipéridone (**22.2**). Un solution de 17,5 g (81 mmol) de 1-fluoro-4-[2-(3-chloropropyloxy)éthyl]benzène (**22.1**) dans 175 ml de DMF, est traitée sous agitation à 25 °C avec 10,4 ml de 1,4-dioxa-8-azaspiro [4,5]décane (11,6 g ou 81 mmol) ; 12,4 g (85 mmol) d'un mélange broyé de K₂CO₃ /KI, 98/02, puis le mélange est chauffé pendant 5 h à 80 °C. L'insoluble est éliminé par filtration et la solution obtenue est évaporé à siccité sous vide. Le résidu est principalement constitué par l'aminocétone protégée de **formule 22.2.1.** qui n'est pas isolée. 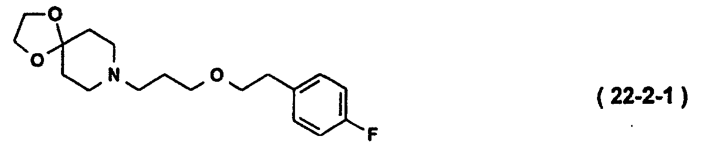 Ce composé est traité avec 160 ml d'acide chlorhydrique 6N et chauffé 2 h à 100° sous agitation. Après retour à 25°, on extrait les insalifiables au CH₂Cl₂ et la phase aqueuse est séparée, refroidie à 0° et alcalinisée avec de la soude 10N jusqu'à pH 12-13. L'amino cétone attendue est extraite au CH₂Cl₂, puis on lave à l'eau, à l'eau salée, sèche sur sulfate de sodium anhydre, filtre et évapore à siccité : obtention de 17,3 g (Rdt : 76 %) de résidu brut de **formule 22.2 :** 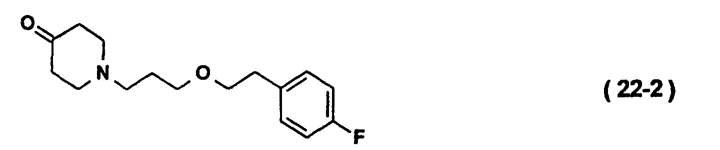 Formule brute : C₁₆H₂₂FNO₂
Masse moléculaire : 279,35
Huile visqueuse légèrement orangée
**RMN** (CDCl₃) δ : 1,5-1,85 (m, 2H) ; 2,36-2,58 (m, 6H) ; 2,72 (t, 4H) ; 2,85 (t, 2H) ; 3,5 (t, 2H) ; 3,61 (t, 2H) ; 6,91-7 (m, 2H) ; 7,1-7,2 (m, 2H).

**22.3)**Dichlorhydrate de la 4-méthylamino-1-[3-[2-(4-fluorophényl)éthoxy]propyl) pipéridine (**22.3**). Une solution de 14,7 g (52,6 mmol) de cétoamine précédente (**22.2**) dans 150 ml de CH₂Cl₂ est traitée avec 3,5 g (52,6 mmol) de chlorhydrate de méthylamine, puis on ajoute du méthanol jusqu'à dissolution totale. Après 3 h d'agitation à 25 °C, le mélange est refroidi sur bain de glace et on ajoute par fraction 14,5 g (68,5 mmol) de triacétate de borohydrure de sodium et enfin 3 ml d'acide acétique goutte à goutte. L'agitation est poursuivie une nuit à 25 °C. Le mélange réactionnel est versé dans de la glace et alcalinisé jusqu'à pH 12-13. La base libérée est extraite de la manière habituelle pour donner une huile verdâtre.
Après dissolution dans 70 ml d'éthanol absolu, et traitement à 0 °C avec une solution éthanolique chlorhydrique 2,5 N, le sel cristallise après amorçage et est récupéré par filtration pour donner 13,6 g (Rdt : 70 %) de cristaux de **formule 22.3 :** 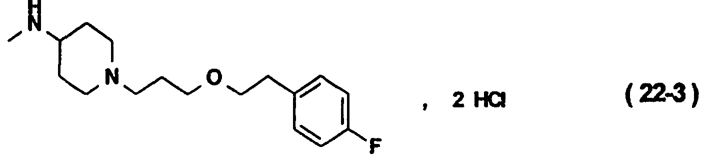 Formule brute : C₁₇H₂₉Cl₂FN₂O
Masse moléculaire : 367,34
Cristaux blanc cassé
**RMN** (DMSO d₆) δ : 1,9-2,04 (m, 4H) ; 2,08-2,28 (m, 2H) ; 2,5 (s, 3H) ; 2,8 (t, 2H) ; 2,85-3,1 (m, 4H) ; 3,17 (m, 1H) ; 3,45 (t, 2H) ; 3,5-3,6 (m, 4H) ; 7,11 (t, 2H) ; 7,25-7,32 (m, 2H) ; 9,3-9,7 (m, 2H) ; 10,6-11,08 (m, 1H).

**22.4)**1-[1-[3-[2-(4-fluorophényl)éthoxy]propyl]pipéridin-4-yl]-1-méthyl-3-phényl thiourée **(22.4).** Une solution de 736 mg (5,44 mmol) d'isothiocyanate de phényle dans 15 ml d'éthanol est traitée avec 2 g (5,44 mmol) de chlorhydrate précédent **22.3,** puis 1,51 ml (10,9 mmoles) de triéthylamine. Après 1 h d'agitation le mélange est évaporé à siccité et traité comme dans l'exemple **1.1** pour donner 1,88 g (Rdt : 81 %) de composé de **formule 22.4 :** 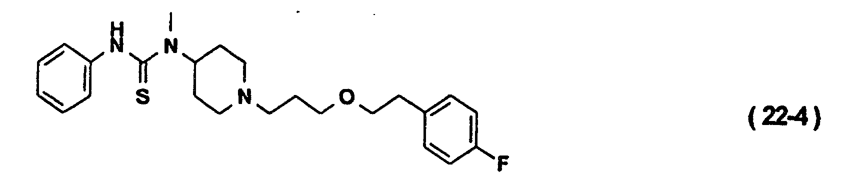 Formule brute : C₂₄H₃₂FN₃OS
Masse moléculaire : 429,58
Poudre blanc cassé
**RMN** (CDCl₃) δ : 1,82-1,97 (m, 4H) ; 1,98-2,18 (m, 2H) ; 2,2-2,41 (m, 2H) ; 2,5-2,68 (m, 2H) ; 2,84 (t, 2H) ; 3,07 (s, 3H) ; 3,08-3,2 (m, 2H) ; 3,48 (t, 2H) ; 3,6 (t, 2H) ; 5,32 (m, 1H) ; 6,97 (t, 2H) ; 7,11 (s, 1H) ; 7,12-7,25 (m, 5H) ; 7,25-7,4 (m, 2H).

**22.5)**Hydrogénofumarate de la 1,2-diméthyl-1-[1-[3-[2-(4-fluorophényl)éthoxy]propyl]pipéridin-4-yl]-3-phényl isothiourée (**22**). En réalisant la S-méthylation de 1,76 g (4,1 mmol) de la thiourée précédente (**22.4**) avec 281 µl (4,51 mmol) d'iodure de méthyle, selon le procédé de l'exemple **1.2,** on prépare 692 mg (Rdt : 30 %) de composé de **formule 22 :** 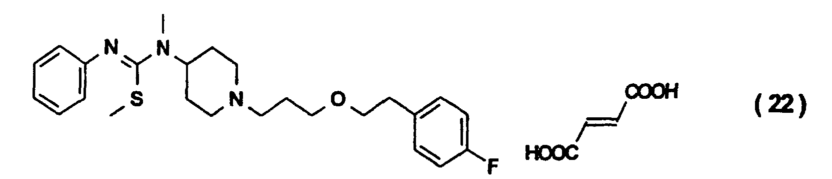 Formule brute : C₂₉H₃₈FN₃O₅S
Masse moléculaire : 559,68
Poudre blanche
Point de fusion : 110-111 °C
**RMN** (DMSO d₆) δ : 1,64-1,72 (m, 4H) ; 1,77-1,91 (m, 2H) ; 2 (s, 3H) ; 2,16 (t, 2H) ; 2,43 (t, 2H) ; 2,78 (t, 2H) ; 2,89 (s, 3H) ; 2,98-3,08 (m, 2H) ; 3,4 (t, 2H) 3,54 (t, 2H) ; 4,1-4,25 (m, 1H) ; 6,58 (s, 2H) ; 6,76 (d, 2H) ; 6,9 (m, 1H) ; 7,09 (m, 2H) ; 7,15-7,3 (m, 4H) ; 13 (m, 2H).

### EXPERIMENTATIONS BIOLOGIQUES :

Les composés de formule I, objets de la présente invention, ainsi que leurs sels thérapeutiquement acceptables possèdent d'intéressantes propriétés pharmacologiques.

Ces dérivés sont actifs au niveau du cardiomyocyte par inhibition de la contracture diastolique induite par la vératrine au niveau de l'oreillette gauche isolée de rat. Ces composés réduisent aussi la contracture diastolique lors de l'ischémie induite sur le coeur isolé perfusé de cobaye sans affecter les propriétés hémodynamiques de base, ce qui en fait des produits fortement sélectifs de l'ischémie.

Ces composés sont aussi actifs *in vivo* lors de l'ischémie-reperfusion chez le lapin anesthésié : ils inhibent les perturbations électriques de l'ECG provoquées par l'ischémie-reperfusion sans effet hémodynamique majeur ; ils ne sont pas dépresseurs cardiaques.

Les molécules de la présente invention sont utiles à titre préventif ou curatif dans le traitement des coronaropathies, de l'angor sous toutes les formes, de l'ischémie cardiaque et cérébrale sous toutes leurs formes et dans le traitement de l'athérosclérose, de l'insuffisance cardiaque, de l'épilepsie, de la douleur et de la migraine.

### 1°) Etude pharmacologique :

Les expérimentations auxquelles ont été soumises les molécules chimiques objet de la présente invention, ont permis de mettre en évidence une activité prometteuse sur le système cardiovasculaire, à la fois par des tests **"*in vitro*"** et **"*in vivo*".**
a) Action **"*in vitro*" :**
- L'inhibition de la contracture à la vératrine, de l'oreillette gauche isolée de rat, a été réalisée selon la technique de **Le Grand** et coll (*Naunyn-Schmiedebergs Arch. Pharmacol*. (1993) **348** p 184-190). Les résultats sont portés dans le tableau **I** ci-après où les pourcentages de l'inhibition sont donnés à la concentration de 10⁻⁷M.

**Tableau I**

| Composé | Ex.1 | Ex.3 | Ex.13 | Ex.21 | R 56865* | Témoin PF** |
|---|---|---|---|---|---|---|
| %.inhibition contracture à 10⁻⁷M | 28% | 32% | 34% | 31% | 33% | 25% |

| | | | | | | |
|---|---|---|---|---|---|---|
| * N-(1-(4-(4-fluorophénoxy)butyl)pipéridin-4-yl)-N-méthyl-2-benzothiazolamine cité dans le brevet EP 0184257. | | | | | | |
| ** N-[1-[4-(3.4-difluorophénoxy)butyl]pipéridin-4-yl]-N-méthyl-4H-3,1-benzothiazin 2-amine décrit dans le brevet WO 97.05134. | | | | | | |

- L'activité antiischémique a été mesurée sur le test du coeur isolé perfusé de cobaye selon la technique de **Le Grand B.** et coll (*Am. J. Physiol*. **269,** H533 - H540, 1995). Les coeurs de cobaye sont prélevés, perfusés par une solution modifiée de Krebs et après 20 minutes, on ajoute le composé à étudier en solution dans l'eau, ou dans un mélange eau - DMSO / 99-01 (selon solubilité). Après 15 minutes l'ischémie globale est provoquée par arrêt de la perfusion coronaire pendant 50 min suivie de la reperfusion durant 1 h. L'inhibition de la contracture ischémique en présence du composé étudié est mesurée par rapport à celle du groupe véhicule. Les résultats sont portés dans le tableau **II** à titre d'exemple non limitatifs à la concentration de 10⁻⁶ M.

**Tableau II**

| Composé | Exemple 1 | Exemple 21 | Témoin PF** |
|---|---|---|---|
| % inhibition de la contracture à 10⁻⁶M | 69% | 53% | 48% |

| | | | |
|---|---|---|---|
| ** voir tableau I. | | | |

b)Activité ***"in vivo"* :**

Les composés de la présente invention sont aussi actifs par voie orale dans le test de **l'ischémie reperfusion chez le lapin anesthésié** selon la méthode de **Verscheure** et coll (*J*. *Cardiovasc Pharmacol.* 1995, **25,** 126-133) . Les résultats pour le compo- sé **1** sont donnés à titre non limitatif dans le tableau **III** suivant :

**Tableau III**

| N° du Produit ou Témoin | Dose mg/kg p.o. | % inhibition surélévation segment ST | Nombre d'animaux présentant des arythmies de reperfusion | % variation fréquence cardiaque | % variation pression artérielle |
|---|---|---|---|---|---|
| 1 | 0,63 | 58% | 1/5 | 5 | -3 |
| Témoin PF** | 0,63 | 33% | 1/5 | 9 | 9 |
| R 56865* | 2,5 | 0% | 2/5 | 0 | 27 |

| | | | | | |
|---|---|---|---|---|---|
| * et ** : voir tableau I. | | | | | |

### 2°) Applications thérapeutiques :

Les composés de la présente invention et leurs sels thérapeutiquement acceptables sont utiles comme médicaments. Ces composés sont plus particulièrement adaptés en cardiologie dans le traitement prophylactique des maladies cardiovasculaires comme :
* l'ischémie du myocarde et les coronaropathies et plus particulièrement dans les crises :
   - d'angor stable chronique,
   - d'angor instable et de Prinzmetal,
* l'ischémie silencieuse, et dans la prévention des réocclusions, resténoses et le réinfarctus ;
* l'ischémie cérébrale et plus précisément dans :
   - l'accident vasculaire cérébral,
   - l'attaque ischémique transitoire,
   - les maladies neurodégénératives,
* l'athérosclérose,
* l'insuffisance cardiaque,
* l'hypertension.

Ces composés peuvent également être utilisés dans le traitement de l'épilepsie, de la migraine et de la douleur. L'administration de ces composés peut être réalisée par voie orale, parentérale ou rectale. Chaque dose est constituée d'un adjuvant inerte favorisant la préparation et l'absorption du médicament ; le principe actif pouvant être aussi associés à un autre.

Ces médicaments peuvent se présenter sous forme solide (comprimés ou gélules) ou liquide à réaliser au moment de l'emploi (suspensions, émulsions, sirops, solutions ou autres) ou suppositoires. L'administration du principe actif se fait à la dose moyenne comprise entre 0,1 et 10 mg/kg du poids du corps.

Deux préparations sont données à titre d'exemple non limitatifs. Les ingrédients ainsi que d'autres, thérapeutiquement acceptables, peuvent être introduits en d'autres proportions sans modifier la portée de l'invention.

### Exemple 23 :

### Solution injectable (à réaliser au moment de l'emploi).

1°) Un flacon stérile pour préparation injectable en verre inactinique renfermant :
Hydrogénofumarate de la 1,2-diméthyl-1-[1-[4-(3,4-difluoro phénoxy)butyl]pipéridin-4-yl]-3-phényl isothiourée ---10 mg

2°) Une ampoule de solvant en verre stérile renfermant

| | |
|---|---|
| Propylène glycol | 80 mg |
| Dextrose anhydre | 40 mg |
| Eau distillée stérile qsp | 2 ml |

### Exemple 24 : comprimés.

Hydrogénofumarate de la 1,2-diméthyl-1-[1-[4-(3,4-difluoro phénoxy)butyl]pipéridin- 4-yl]-3-phényl isothiourée - 30 mg

| | |
|---|---|
| Lactose hydrate | 90 mg |
| Cellulose microcristalline | 20 mg |
| Stéarate de magnésium | 5 mg |
| Amidon de maïs | 20 mg |
| Talc | 3 mg |
| Polyvinyl pyrrolidone | 7 mg |
| Poids total | 175 mg |

Comprimés sécables à conserver à l'abri de la chaleur et de l'humidité.

## Revendications

1. 1,2-alcoyl-1-[1-(aryl(alcoyl)oxyalcoyl) pipéridin-4-yl]-3-aryl isothiourées de formule **I** : 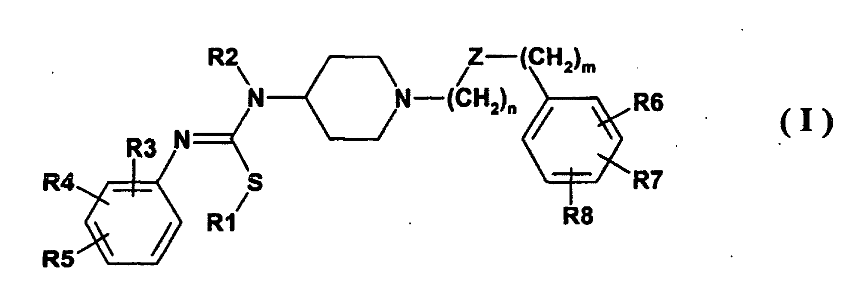 dans laquelle :
R₁ et R₂ égaux ou différents représentent un radical alcoyle saturé ou non, ramifié ou non renfermant de 1 à 7 atomes de carbone,
**R**_{**3**} **à R**_{**8**} égaux ou différents représentent :
- un hydrogène
- un alcoyle ramifié ou non renfermant 1 à 5 atomes de carbone
- un alcoyloxy ramifié ou non renfermant 1 à 5 atomes de carbone
- un groupement halogéno
- un groupement nitro
- un groupement hydroxy
- un groupement acyle ou acyloxy comportant 1 à 5 atomes de carbone
- un groupement dialcoylamino renfermant 1 à 5 atomes de carbone
- un groupement trifluorométhyle ou trifluoro méthoxyle ;
**Z** représente un atome d'oxygène ou de soufre ou un méthylène ;
**m** représente un nombre entier variant de 0 à 4 inclus ;
n représente un nombre entier compris entre 2 et 7 inclus.
ainsi que leurs énantiomères purs et leurs mélanges, et les sels minéraux ou organiques thérapeutiquement acceptables des composés de formule I et leurs hydrates éventuels.

2. Composé selon la revendication 1 **caractérisé en ce qu'**il est choisi parmi les composés suivants :
Hydrogénofumarate de la 1,2-diméthyl-3-phényl-1-[1-[4-(3,4-difluorophénoxy)butyl] piperidin-4-yl] isothiourée.
Hydrogénofumarate de la 2-éthyl-1-[1-[4-(3,4-difluorophénoxy)butyl]pipéridin-4-yl]-1-méthyl-3-phényl isothiourée.
Hydrogénofumarate de la 1-éthyl-1-[1-[4-(3,4-difluorophénoxy)butyl]piperidin-4-yl]-2-méthyl-3-phényl isothiourée.
Hydrogénofumarate de la 1-isobutyl-1-[1-[4-(3,4-difluorophénoxy)butyl]pipéridin-4-yl]-2-méthyl-3-phényl isothiourée.
Hydrogénofumarate de la 1,2-diméthyl-1-[1-[5-(3,4-difluorophénoxy)pentyl] pipéridin -4-yl]-3-phényl isothiourée.
Hydrogénofumarate de la 1,2-diméthyl-1-[1-[3-(3,4-difluorophénoxy)propyl]pipérin-4-yl]-3-phényl isothiourée.
Hydrogénofumarate de la 1,2-diméthyl-1-[1-[4-(4-fluorophénoxy)butyl]pipéridin-4-yl]-3-phényl isothiourée.
Hydrogénofumarate de la 1,2-diméthyl-1-[1-[2-(4-fluorophénoxy)éthyl]pipéridin-4-yl]-3-phényl isothiourée.
Hydrogénofumarate de la 1,2-diméthyl-1-[1-[4-(4-méthoxyphénoxy)butyl]pipéridin-4-yl] -3-phényl isothiourée.
Hydrogénofumarate de la 1,2-diméthyl-1-[1-[4-(3,4-diméthylphénoxy)butyl] pipéridin -4-yl]-3-phényl isothiourée.
Hydrogénofumarate de la 1,2-diméthyl-1-[1-[4-(2-méthoxy-4-chloro phénoxy)butyl] pipéridin-4-yl]-3-phényl isothiourée.
Hydrogénofumarate de la 1,2-diméthyl-1-[1-[4-(3,4-difluorophénoxy)butyl] pipéridin -4-yl]-3-(2-fluorophényl) isothiourée.
Hydrogénofumarate de la 1,2-diméthyl-1-[1-[4-(3,4-difluorophénoxy)butyl]pipéridin -4-yl]-3-(4-fluorophényl) isothiourée.
Hydrogénofumarate de la 1,2-diméthyl-1-[1-[4-(3,4-difluorophénoxy)butyl]pipéridin -4-yl]-3-(4-chlorophényl) isothiourée.
Hydrogénofumarate de la 1,2-diméthyl-1-[1-[4-(3,4-difluorophénoxy)butyl]pipéridin -4-yl]-3-(4-méthylphényl) isothiourée.
Hydrogénofumarate de la 1,2-diméthyl-1-[1-[4-(3,4-difluorophénoxy)butyl)pipéridin-4-yl]-3-(4-méthoxyphényl) isothiourée.
Hydrogénofumarate de la 1,2-diméthyl-1-[1-[4-(3,4-difluorophénoxy)butyl]pipéridin -4-yl]-3-(2,6-diméthylphényl) isothiourée.
Hydrogénofumarate de la 1,2-diméthyl-1-[1-[4-(3,4-difluorophénoxy)butyl]pipéridin -4-yl]-3-[2,6-diisopropylphényl] isothiourée.
Hydrogénofumarate de la 1,2-diméthyl-1-[1-[4-(3,4-difluorophénoxy)butyl]pipéridin-4-yl]-3-(3-fluorophényl) isothiourée.
Hydrogénofumarate de la 1,2-diméthyl-1-[1-[4-(3,4-difluorophénoxy)butyl]pipé-ridin-4-yl]-3-(4-nitrophényl) isothiourée.
Hydrogénofumarate de la 1,2-diméthyl-1-[1-[4-(3,4-difluorophénoxy)butyl]pipéridin -4-yl]-3-(2,6-difluorophényl) isothiourée.
Hydrogénofumarate de la 1,2-diméthyl-1-[1-[3-[2-(4-fluorophényl)éthoxy]propyl] pipéridin-4-yl]-3-phényl isothiourée.

3. Procédé de préparation des composés chimiques de formule I selon les revendications 1 et 2, **caractérisé en ce que** l'on fait réagir un isothiocyanate de formule **(III) :** 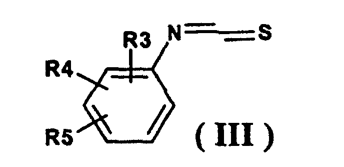 sur une diamine de formule **(II)** : 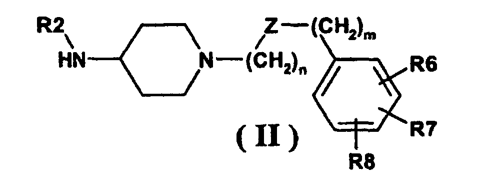 dans un solvant protique comme l'éthanol ou aprotique comme le THF ou le dioxane en chauffant ou non au reflux pour donner la thiourée intermédiaire de formule **(IV)** : 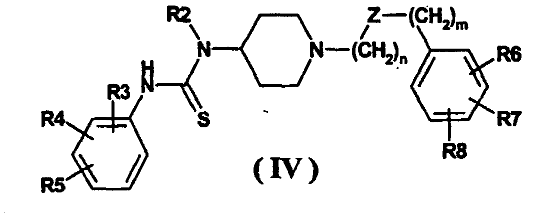 qui est ensuite S-alcoylée avec un halogénure R₁Br, R₁I, ou un sulfate (R₁)₂SO₄ d'alcoyle dans un solvant protique comme l'alcool ou aprotique comme le THF, DMF, ou l'acétate d'éthyle en présence ou non de chaux à une température comprise entre 20 et 60 °C pour donner le composé **I** de la présente invention. Dans les formules **II, III,** et **IV,** les radicaux **R**_{**1**} à **R**_{**8**}**, Z** et les nombres **m** et **n** ont les mêmes valeurs que dans la revendication 1.

4. Les composés selon l'une des revendications 1 et 2 pour utilisation comme médicaments.

5. Composition pharmaceutique **caractérisée en ce qu'**elle contient comme principe actif au moins un composé selon l'une des revendications 1 et 2, associé à un support pharmaceutique inerte, ou autres véhicules pharmaceutiquement acceptables et pouvant ou non être associé à un autre médicament.

6. Composition pharmaceutique selon la revendication 5 utile dans le traitement prophylactique de l'ischémie myocardique comme les crises d'angor stable chronique, l'angor instable et de Prinzmetal, l'ischémie silencieuse, le réinfarctus, la réocclusion et la resténose.

7. Composition pharmaceutique selon la revendication 5, utile dans l'ischémie cérébrale, l'accident vasculaire cérébral, l'attaque ischémique transitoire et les maladies neurodégénératives.

8. Composition pharmaceutique selon la revendication 5, **caractérisée en ce qu'**elle permet de prévenir et de traiter l'athérosclérose.

9. Composition pharmaceutique selon la revendication 5, **caractérisée en ce qu'**elle permet de traiter l'insuffisance cardiaque et l'hypertension.

10. Composition pharmaceutique selon la revendication 5, **caractérisée en ce qu'**elle permet de traiter l'épilepsie, la migraine et la douleur.

## Patentansprüche

1. 1,2-Alkyl-1-[1-(aryl(alkyl)oxyalkyl)piperidin-4-yl]-3-arylisothioharnstoffe der Formel I: 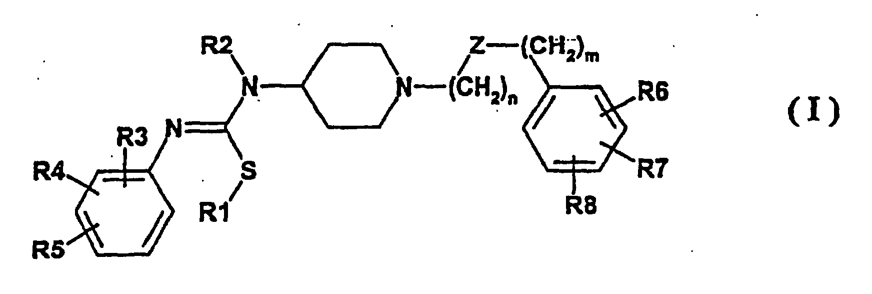 in der:
R₁ und R₂, gleich oder verschieden, einen gesättigten oder ungesättigten, verzweigten oder unverzweigten Alkylrest darstellen, der 1 bis 7 Kohlenstoffatome einschließt,
R₃ bis R₈, gleich oder verschieden, darstellen:
- einen Wasserstoff
- ein verzweigtes oder unverzweigtes Alkyl, das 1 bis 5 Kohlenstoffatome einschließt
- ein verzweigtes oder unverzweigtes Alkyloxy, das 1 bis 5 Kohlenstoffatome einschließt
- eine Halogen-Gruppe
- eine Nitro-Gruppe
- eine Hydroxy-Gruppe
- eine Acyl- oder Acyloxy-Gruppe, die 1 bis 5 Kohlenstoffatome umfaßt
- eine Dialkylamino-Gruppe, die 1 bis 5 Kohlenstoffatome einschließt
- eine Trifluormethyl- oder Trifluormethoxy-Gruppe;
Z ein Sauerstoff- oder Schwefelatom oder ein Methylen darstellt;
m eine ganze Zahl einschließt, die von 0 bis 4 einschließlich variiert;
n eine ganze Zahl darstellt, die von 2 bis 7 einschließlich variiert,
sowie deren reine Enantiomere und deren Mischungen und die therapeutisch annehmbaren Mineral- oder organischen Salze der Verbindungen der Formel I und ihre eventuellen Hydrate.

2. Verbindung nach Anspruch 1, **dadurch gekennzeichnet, daß** sie aus den folgenden Verbindungen ausgewählt ist:
1,2-Dimethyl-3-phenyl-1-[1-[4-(3,4-difluorphenoxy)butyl]piperidin-4-yl]isothioharnstoff-Hydrogenfumarat;
2-Ethyl-1-[1-[4-(3,4-difluorphenoxy)butyl]piperidin-4-yl]-1-methyl-3-phenylisothioharnstoff-Hydrogenfumarat;
1-Ethyl-1-[1-[4-(3,4-difluorphenoxy)butyl]piperidin-4-yl]-2-methyl-3-phenylisothioharnstoff-Hydrogenfumarat;
1-Isobutyl-1-[1-[4-(3,4-difluorphenoxy)butyl]piperidin-4-yl]-2-methyl-3-phenylisothioharnstoff-Hydrogenfumarat:
1,2-Dimethyl-1-[1-[5-(3,4-difluorphenoxy)pentyl]piperidin-4-yl]-3-phenylisothioharnstoff-Hydrogenfumarat;
1,2-Dimethyl-1-[1-[3-(3,4-difluorphenoxy)propyl]piperidin-4-yl]-3-phenylisothioharnstoff-Hydrogenfumarat;
1,2-Dimethyl-1-[1-[4-(4-fluorphenoxy)butyl]piperidin-4-yl]-3-phenylisothioharnstoff-Hydrogenfumarat;
1,2-Dimethyl-1-[1-[2-(4-fluorphenoxy)ethyl]piperidin-4-yl]-3-phenylisothioharnstoff-Hydrogenfumarat;
1,2-Dimethyl-1-[1-[4-(4-methoxyphenoxy)butyl]piperidin-4-yl]-3-phenylisothioharnstoff-Hydrogenfumarat;
1,2-Dimethyl-1-[1-[4-(3,4-dimethylphenoxy)butyl]piperidin-4-yl]-3-phenylisothioharnstoff-Hydrogenfumarat;
1,2-Dimethyl-1-[1-[4-(2-methoxy-4-chlorphenoxy)butyl]piperidin-4-yl]-3-phenylisothioharnstoff-Hydrogenfumarat;
1,2-Dimethyl-1-[1-[4-(3,4-difluorphenoxy)butyl]piperidin-4-yl]-3-(2-fluorphenyl)isothioharnstoff-Hydrogenfumarat;
1,2-Dimethyl-1-[1-[4-(3,4-difluorphenoxy)butyl]piperidin-4-yl]-3-(4-fluorphenyl)isothioharnstoff-Hydrogenfumarat;
1,2-Dimethyl-1-[1-[4-(3,4-difluorphenoxy)butyl]piperidin-4-yl]-3-(4-chlorphenyl)isothioharnstoff-Hydrogenfumarat;
1,2-Dimethyl-1-[1-[4-(3,4-difluorphenoxy)butyl]piperidin-4-yl]-3-(4-methylphenyl)-isothioharnstoff-Hydrogenfumarat;
1,2-Dimethyl-1-[1-[4-(3,4-difluorphenoxy)butyl]piperidin-4-yl]-3-(4-methoxyphenyl)-isothioharnstoff-Hydrogenfumarat;
1,2-Dimethyl-1-[1-[4-(3,4-difluorphenoxy)butyl]piperidin-4-yl]-3-(2,6-dimethylphenyl)isothioharnstoff-Hydrogenfumarat;
1,2-Dimethyl-1-[1-[4-(3,4-difluorphenoxy)butyl]piperidin-4-yl]-3-[2,6-diisopropylphenyl]isothioharnstoff-Hydrogenfumarat;
1,2-Dimethyl-1-[1-[4-(3,4-difluorphenoxy)butyl]piperidin-4-yl]-3-(3-fluorphenyl)isothioharnstoff-Hydrogenfumarat;
1,2-Dimethyl-1-[1-[4-(3,4-difluorphenoxy)butyl]piperidin-4-yl]-3-(4-nitrophenyl)isothioharnstoff-Hydrogenfumarat;
1,2-Dimethyl-1-[1-[4-(3,4-difluorphenoxy)butyl]piperidin-4-yl]-3-(2,6-difluorphenyl)-isothioharnstoff-Hydrogenfumarat;
1,2-Dimethyl-1-[1-[3-[2-(4-fluorphenyl)ethoxy]propyl]piperidin-4-yl]-3-phenylisothioharnstoff-Hydrogenfumarat.

3. Verfahren zur Herstellung von chemischen Verbindungen der Formel I nach den Ansprüchen 1 und 2, **dadurch gekennzeichnet, daß** man ein Isothiocyanat der Formel (III): 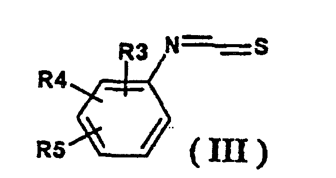 mit einem Diamin der Formel (II): 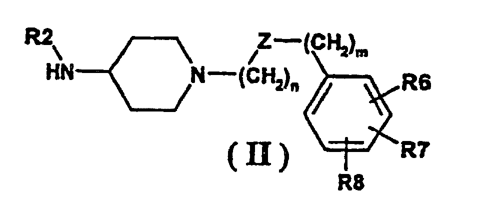 in einem protischen Lösungsmittel wie Ethanol oder einem aprotischen Lösungsmittel wie THF oder Dioxan reagieren läßt, indem man zum Rückfluß erwärmt oder nicht, um das Thioharnstoff-Zwischenprodukt der Formel (IV) zu ergeben: 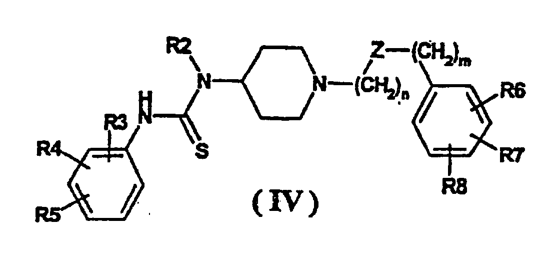 das anschließend mit―einem Alkylhalogenid R₁Br, R₁I oder einem Alkylsulfat (R₁)₂SO₄ in einem protischen Lösungsmittel wie Alkohol oder einem aprotischen Lösungsmittel wie THF, DMF oder Ethylacetat in Anwesenheit oder nicht von Wärme bei einer Temperatur zwischen 20 und 60 °C S-alkyliert wird, um die Verbindung I der vorliegenden Erfindung zu ergeben; wobei in den Formeln II, III und IV die Reste R₁ bis R₈, Z und die Zahlen m und n die gleichen Bedeutungen aufweisen wie in Anspruch 1.

4. Die Verbindungen nach einem der Ansprüche 1 und 2 zur Verwendung als Medikamente.

5. Pharmazeutische Zusammensetzung, **dadurch gekennzeichnet, daß** sie als Wirkstoff mindestens eine Verbindung nach einem der Ansprüche 1 und 2 in Verbindung mit einem inerten pharmazeutischen Träger oder anderen pharmazeutisch annehmbaren Vehikeln enthält und mit einem weiteren Medikament assoziiert sein kann oder nicht.

6. Pharmazeutische Zusammensetzung nach Anspruch 5, die bei der prophylaktischen Behandlung von Myocardischämie, wie stabilen chronischen Herzbräune-Krisen, instabiler und Prinzmetal-Angina, stummer Ischämie, Reinfarkt, Reokklusion und Restenose, nützlich ist.

7. Pharmazeutische Zusammensetzung nach Anspruch 5, die bei Zerebralischämie, zerebralem vaskulärem Insult, vorübergehender ischämischer Attacke und neurodegenerativen Erkrankungen nützlich ist.

8. Pharmazeutische Zusammensetzung nach Anspruch 5, **dadurch gekennzeichnet, daß** sie die Verhütung und Behandlung von Atherosklerose ermöglicht.

9. Pharmazeutische Zusammensetzung nach Anspruch 5, **dadurch gekennzeichnet, daß** sie die Behandlung von Herzinsuffizienz und Bluthochdruck ermöglicht.

10. Pharmazeutische Zusammensetzung nach Anspruch 5, **dadurch gekennzeichnet, daß** sie die Behandlung von Epilepsie, Migräne und Schmerzen ermöglicht.

## Claims

1. 1,2-Dialkyl-1-[1-(aryl(alkyl)oxyalkyl)-piperidin-4-yl]-3-arylisothioureas of formula **I:** 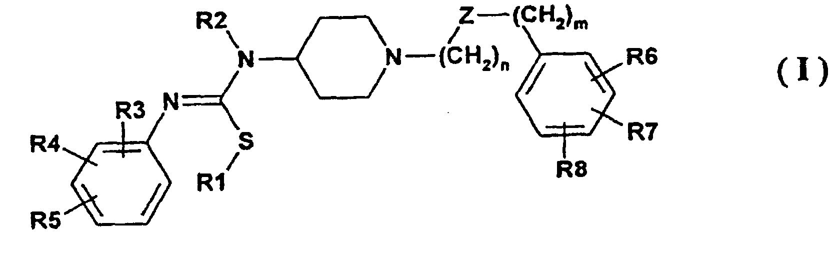 in which:
**R**_{**1**} and **R**_{**2**}**,** which are identical or different, represent a saturated or unsaturated and branched or unbranched alkyl radical having from 1 to 7 carbon atoms,
**R**_{**3**} to **R**_{**8**}**,** which are identical or different, represent:
- a hydrogen,
- a branched or unbranched alkyl having 1 to 5 carbon atoms,
- a branched or unbranched alkyloxy having 1 to 5 carbon atoms,
- a halo group,
- a nitro group,
- a hydroxyl group,
- an acyl or acyloxy group comprising 1 to 5 carbon atoms,
- a dialkylamino group having 1 to 5 carbon atoms,
- a trifluoromethyl or trifluoromethoxyl group,
**Z** represents an oxygen or sulphur atom or a methylene,
m represents an integer varying from 0 to 4 inclusive,
**n** represents an integer of between 2 and 7 inclusive;
and their pure enantiomers and their mixtures, and the therapeutically acceptable organic or inorganic salts of the compounds of formula I and their possible hydrates.

2. Compound according to Claim 1, **characterized in that** it is chosen from the following compounds:
1,2-Dimethyl-3-phenyl-1-[1-[4-(3,4-difluorophenoxy)butyl]piperidin-4-yl]isothiourea hydrogen fumarate.
2-Ethyl-1-[1-[4-(3,4-difluorophenoxy)butyl]piperidin-4-yl]-1-methyl-3-phenylisothiourea hydrogen fumarate.
1-Ethyl-1-[1-[4-(3,4-difluorophenoxy)butyl]piperidin-4-yl]-2-methyl-3-phenylisothiourea hydrogen fumarate.
1-Isobutyl-1-[1-[4-(3,4-difluorophenoxy)butyl]piperidin-4-yl]-2-methyl-3-phenylisothiourea hydrogen fumarate.
1,2-Dimethyl-1-[1-[5-(3,4-difluorophenoxy)pentyl]piperidin-4-yl]-3-phenylisothiourea hydrogen fumarate.
1,2-Dimethyl-1-[1-[3-(3,4-difluorophenoxy)propyl]piperidin-4-yl]-3-phenylisothiourea hydrogen fumarate.
1,2-Dimethyl-1-[1-[4-(4-fluorophenoxy)butyl]piperidin-4-yl]-3-phenylisothiourea hydrogen fumarate.
1,2-Dimethyl-1-[1-[2-(4-fluorophenoxy)ethyl]piperidin-4-yl]-3-phenylisothiourea hydrogen fumarate.
1,2-Dimethyl-1-[1-[4-(4-methoxyphenoxy)butyl]piperidin-4-yl]-3-phenylisothiourea hydrogen fumarate.
1,2-Dimethyl-1-[1-[4-(3,4-dimethylphenoxy)butyl]piperidin-4-yl]-3-phenylisothiourea hydrogen fumarate.
2,2-Dimethyl-1-[1-[4-(2-methoxy-4-chlorophenoxy)butyl]piperidin-4-yl]-3-phenylisothiourea hydrogen fumarate.
1,2-Dimethyl-1-[1-[4-(3,4-difluorophenoxy)butyl]piperidin-4-yl]-3-(2-fluorophenyl)isothiourea hydrogen fumarate.
1,2-Dimethyl-1-[1-[4-(3,4-difluorophenoxy)butyl]piperidin-4-yl]-3-(4-fluorophenyl)isothiourea hydrogen fumarate.
1,2-Dimethyl-1-[1-[4-(3,4-difluorophenoxy)butyl]piperidin-4-yl]-3-(4-chlorophenyl)isothiourea hydrogen fumarate.
1,2-Dimethyl-1-[1-[4-(3,4-difluorophenoxy)butyl]piperidin-4-yl]-3-(4-methylphenyl)isothiourea hydrogen fumarate.
1,2-Dimethyl-1-[1-[4-(3,4-difluorophenoxy)butyl]piperidin-4-yl]-3-(4-methoxyphenyl)isothiourea hydrogen fumarate.
1,2-Dimethyl-1-[1-[4-(3,4-difluorophenoxy)butyl]piperidin-4-yl]-3-(2,6-dimethylphenyl)isothiourea hydrogen fumarate.
1,2-Dimethyl-1-[1-[4-(3,4-difluorophenoxy)butyl]piperidin-4-yl]-3-[2,6-diisopropylphenyl]isothiourea hydrogen fumarate.
1,2-Dimethyl-1-[1-[4-(3,4-difluorophenoxy)butyl]piperidin-4-yl]-3-(3-fluorophenyl)isothiourea hydrogen fumarate.
1,2-Dimethyl-2-[1-[4-(3,4-difluorophenoxy)butyl]piperidin-4-yl]-3-(4-nitrophenyl)isothiourea hydrogen fumarate.
1,2-Dimethyl-1-[1-[4-(3,4-difluorophenoxy)butyl]piperidin-4-yl]-3-(2,6-difluorophenyl)isothiourea hydrogen fumarate.
1,2-Dimethyl-1-[1-[3-[2-(4-fluorophenyl)ethoxy]propyl]piperidin-4-yl]-3-phenylisothiourea hydrogen fumarate.

3. Process for the preparation of the chemical compounds of formula I according to Claims 1 and 2, **characterized in that** an isothiocyanate of formula **(III):** 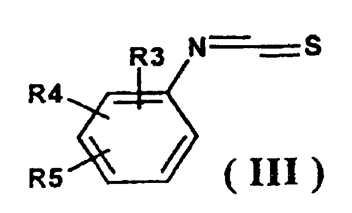 is reacted with a diamine of formula (**II**): 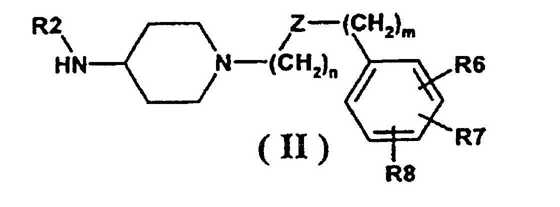 in a protic solvent, such as ethanol, or an aprotic solvent, such as THF or dioxane, by heating or not heating at reflux, to give the intermediate thiourea of formula **(IV):** 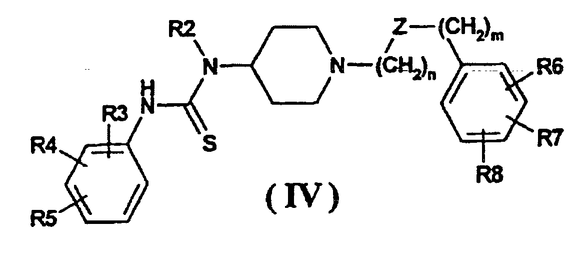 which is subsequently S-alkylated with a halide R₁Br or R₁I or an alkyl sulphate (R₁)₂SO₄ in a protic solvent, such as alcohol, or an aprotic solvent, such as THF, DMF or ethyl acetate, in the presence or in the absence of lime at a temperature of between 20 and 60°C, to give the compound **I** of the present invention.
In the formulae **II, III** and **IV,** the **R**_{**1**} to **R**_{**8**} and **Z** radicals and the numbers **m** and **n** have the same values as in Claim 1.

4. The compounds according to either of Claims 1 and 2, for use as medicaments.

5. Pharmaceutical composition, **characterized in that** it comprises, as active principle, at least one compound according to either of Claims 1 and 2 in combination with an inert pharmaceutical excipient or other pharmaceutically acceptable vehicles which may or may not be used in combination with another medicament.

6. Pharmaceutical composition according to Claim 5, of use in the prophylactic treatment of myocardial ischaemia, such as crises of chronic stable angina, unstable and Prinzmetal's angina, silent ischaemia, reinfarctus, reocclusion and restenosis.

7. Pharmaceutical composition according to Claim 5, of use in cerebral ischaemia, strokes, transitory ischaemic attack and neurodegenerative diseases.

8. Pharmaceutical composition according to Claim 5, **characterized in that** it makes it possible to prevent and to treat atherosclerosis.

9. Pharmaceutical composition according to Claim 5, **characterized in that** it makes it possible to treat cardiac insufficiency and hypertension.

10. Pharmaceutical composition according to Claim 5, **characterized in that** it makes it possible to treat epilepsy, migraine and pain.
